# EUROPEAN PATENT APPLICATION

(11) **EP 3 358 570 A1**
(43) Date of publication of application: **08.08.2018**
(21) Application number: 15905334.7
(22) Date of filing: 29.09.2015
(51) Int. Cl.: G16H 10/00

(54) **PROGRAM, INFORMATION PROCESSING METHOD, AND INFORMATION PROCESSING DEVICE**

(71) Applicant: Fujitsu Limited, Kawasaki-shi, Kanagawa 211-8588 (JP)
(72) Inventor: HOTTA, Shinji, Kawasaki-shi Kanagawa 211-8588 (JP); INOMATA Akihiro, Hayes, Middlesex UB4 8FE (GB)
(74) Representative: Haseltine Lake LLP
(86) International application number: PCT/JP2015/077453
(87) International publication number: WO 2017/056179

(57) **Abstract**

To provide a program or the like that efficiently extracts combinations of related events, from time-series data recorded with event times of a large number of events.

The program causes a computer 20 to execute a process which includes acquiring time-series data including information related to types of events and times when the events are generated, and dividing the time-series data into groups at positions where an interval between a time when one event is generated to a time when an event next to the one event is generated, is longer than a predetermined time by which the events are regarded as having causality therebetween.

## Description

### TECHNICAL FIELD

The present invention relates to a program an information processing apparatus, and an information processing method.

### BACKGROUND ART

A computer network or the like is used to collect time-series data recorded with event times of a large number of events. A disclosed method combines time intervals between the events and generation patterns of the events, to divide the time-series data into a plurality of series (Patent Document 1).

A disclosed method extracts the time-series data using a window with a fixed length, counts combinations of the events within the window, and thereafter repeats a process of shifting the window by a predetermined time, to identify combinations of mutually related events included within the time-series data (Non-Patent Document 1).

A disclosed method counts combinations of a head event of the window and other events within the window, and thereafter repeats a process of shifting a start position of the window to a second event, to identify the combinations of mutually related events included within the time-series data' (Non-Patent Document 2).

### PRIOR ART DOCUMENTS

### PATENT DOCUMENTS

Patent Document 1: Japanese Laid-Open Patent Publication No. 2010-61412

### NON-PATENT DOCUMENTS

Non-Patent Document 1: H. Mannila, "Discovery of Frequent Episodes in Event Sequences", Data Mining and Knowledge Discovery, 1997, Vol. 1, pp. 259-289
Non-Patent Document 2: A. Tang, "Efficient Mining of Intertransaction Association Rules", IEEE Transactions on Knowledge and Data Engineering, 2003, Vol. 15, pp. 43-56

### DISCLOSURE OF THE INVENTION

### PROBLEM TO BE SOLVED BY THE INVENTION

The method of the Patent Document 1 is the method of dividing the time-series data, and cannot identify the combinations of the mutually related events. The methods of the Non-Patent Document 1 and the Non-Patent Document 2 require a long computation time in a case in which an amount of the time-series data is large, because it takes time to perform the operation of shifting the window.

One object according to one aspect is to provide a program or the like for efficiently extracting the combinations of related events, from the time-series data recorded with the event times of a large number of events.

### MEANS OF SOLVING THE PROBLEM

According to one aspect of the present invention, a program causes a computer to execute a process that includes acquiring time-series data including information related to types of events and times when the events are generated, and dividing the time-series data into groups at positions where an interval between a time when one event is generated to a time when an event next to the one event is generated, is longer than a predetermined time by which the events are regarded as having causality therebetween.

### EFFECTS OF THE INVENTION

According to one aspect, it is possible to provide a program or the like that efficiently extracts the combinations of related events, from the time-series data recorded with the event times of a large number of events.

### BRIEF DESCRIPTION OF THE DRAWING

FIG. 1 is a schematic diagram illustrating a configuration of an information processing system;
FIG. 2 is a schematic diagram illustrating configurations of a server and a client;
FIG. 3 is a schematic diagram illustrating a record layout of an event DB;
FIG. 4 is a schematic diagram illustrating a record layout of a time-series DB;
FIG. 5 is a schematic diagram illustrating a record layout of a combination count DB;
FIG. 6 is a schematic diagram illustrating a record layout of a combination time DB;
FIG. 7 is a schematic diagram illustrating a record layout of a generation number DB;
FIG. 8 is a schematic diagram illustrating a record layout of a combination probability DB;
FIG. 9 is a schematic diagram illustrating a record layout of a window DB;
FIG. 10 is a schematic diagram illustrating an example of generation state of events;
FIG. 11 is a schematic diagram illustrating the example of the generation state of the events;
FIG. 12 is a schematic diagram illustrating the example of the generation state of the events;
FIG. 13 is a schematic diagram illustrating the example of the generation state of the events;
FIG. 14 is a flow chart illustrating a processing flow of a program;
FIG. 15 is a flow chart illustrating a processing flow of a subroutine for group acquisition;
FIG. 16 is a flow chart illustrating a processing flow of a subroutine for complete isolation count;
FIG. 17 is a flow chart illustrating a processing flow of a subroutine for semi-isolation count;
FIG. 18 is a flow chart illustrating a processing flow of a subroutine for creating a window DB;
FIG. 19 is a flow chart illustrating the processing flow of the subroutine for creasing the window DB;
FIG. 20 is a schematic diagram illustrating a screen that displays results of information processing;
FIG. 21 is a schematic diagram illustrating the screen that displays the results of the information processing;
FIG. 22 is a schematic diagram illustrating the screen that displays the results of the information processing;
FIG. 23 is a flow chart illustrating a processing flow of a program of a comparison example;
FIG. 24 is a schematic diagram illustrating effects of reducing computation time;
FIG. 25 is a schematic diagram illustrating the effects of reducing the computation time;
FIG. 26 is a schematic diagram illustrating a record layout of the time-series DB in an embodiment 2;
FIG. 27 is a flow chart illustrating a processing flow of a program in the embodiment 2;
FIG. 28 is a flow chart illustrating the processing flow of the program in the embodiment 2;
FIG. 29 is a schematic diagram illustrating an example of generation state of events in an embodiment 3;
FIG. 30 is a schematic diagram illustrating the example of the generation state of the events in the embodiment 3;
FIG. 31 is a schematic diagram illustrating a record layout of a group DB in the embodiment 3;
FIG. 32 is a schematic diagram illustrating the record layout of the group DB in the embodiment 3;
FIG. 33 is a flow chart illustrating a processing flow of a program in the embodiment 3;
FIG. 34 is a flow chart illustrating the processing flow of the program in the embodiment 3;
FIG. 35 is a flow chart illustrating a processing flow of a subroutine for group division in the embodiment 3;
FIG. 36 is a flow chart illustrating a processing flow of a subroutine for boundary flag insertion in the embodiment 3;
FIG. 37 is a flow chart illustrating a processing flow of a subroutine for counting before and after a boundary flag in the embodiment 3;
FIG. 38 is a schematic diagram illustrating an example of a generation state of events in an embodiment 4;
FIG. 39 is a schematic diagram illustrating a record layout of a time difference addition DB in the embodiment 4;
FIG. 40 is a schematic diagram illustrating the record layout of the time difference addition DB in the embodiment 4;
FIG. 41 is a flow chart illustrating a processing flow of a subroutine for group division in the embodiment 4;
FIG. 42 is a flow chart illustrating a processing flow of a subroutine for boundary count computation in the embodiment 4;
FIG. 43 is a schematic diagram illustrating an example of generation state of events in an embodiment 5;
FIG. 44 is a schematic diagram illustrating the example of the generation state of the events in the embodiment 5;
FIG. 45 is a schematic diagram illustrating the example of the generation state of the events in the embodiment 5;
FIG. 46 is a schematic diagram illustrating a record layout of a group DB in the embodiment 5;
FIG. 47 is a flow chart illustrating a processing flow of a subroutine for group division in the embodiment 5;
FIG. 48 is a flow chart illustrating a processing flow of a subroutine for boundary flag insertion in the embodiment 5;
FIG. 49 is a schematic diagram illustrating a record layout of a combination count DB in an embodiment 6;
FIG. 50 is a flow chart illustrating a processing flow of a program in the embodiment 6;
FIG. 51 is a functional block diagram illustrating operations of an information processing apparatus in an embodiment 7; and
FIG. 52 is a schematic diagram illustrating a configuration of an information processing system in an embodiment 8.

### MODE OF CARRYING OUT THE INVENTION

### [Embodiment 1]

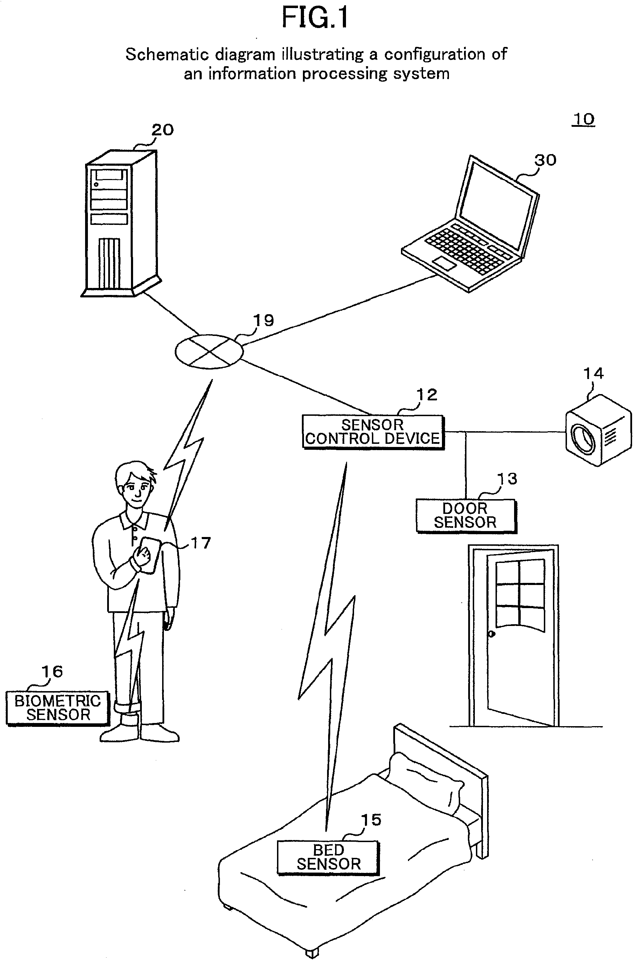

FIG. 1 is a schematic diagram illustrating a configuration of an information processing system 10. The information processing system 10 includes a server 20, a client 30, a sensor control device 12, a portable information device 17, and a network 19. A biometric sensor 16 is connected to the portable information device 17 by a wireless connection. A door sensor 13, a camera 14, and a head sensor 15 are connected to the sensor control device 12. The server 20 is an example of an information processing apparatus of this embodiment. Configurations of the server 20 and the client 30 will be described later.

The network 19 is an information communication network, such as the Internet, a public switched network, a private line network, or the like, for example. The portable information device 17 is an information device such as a smartphone, a mobile phone, wearable computer, or the like carried by a subject. The portable information device 17 is connected to a network through a wireless LAN (Local Area Network) or a mobile communication line. The biometric sensor 16 is a sensor that measures a pulse, a blood oxygen level, a respiratory rate, or the like of the subject. A plurality of biometric sensors 16 may be connected to the portable information device 17. In addition, the biometric sensor 16, such as a pedometer or the like, may be built into the portable information device 17.

The door sensor 13 is a sensor that detects opening and closing of a door, or detects a person passing through an open door. The door sensor 13 is mounted on each door in a subject's house, such as a door of a bedroom of the subject, or the like. The camera 14 is a sensor that detects movements of the subject using video. The camera 14 is mounted at various locations in the subject's house, such as in the bedroom of the subject, or the like. The bed sensor 15 is a sensor that detects a state of the subject during sleep. The bed sensor 15 is mounted on the bed of the subject. Various kinds of sensors, including a thermometer that detects a temperature within a room, a hygrometer that detects a humidity within the room, an illuminometer that detects a luminosity within the room, or the like, may be connected to the sensor control device 12. In addition, sensors that detect operation states of various devices within the house, such as an air conditioner, a lighting device, or the like, may be connected to the sensor control device 12. Further, the sensors may be connected directly to the network 19.

The sensor control device 12 and the portable information device 17 analyze information acquired from each of the connected sensors to obtain a change in state of the subject, and transmit the change in the state of the subject to the server 20 via the network 19. An example of the information that is transmitted will be described later. The sensor control device 12 may be connected to the information processing 10 at a plurality of locations, such as the subject's house, a workplace, or the like, for example.

For example, information that indicates passing through an automatic ticket gate using an IC card, information that indicates shopping using the IC card, or the like may be transmitted from a server of an operating company of the IC card to the server 20 via the network 19. For example, information that is obtained by analyzing, by a medical institution, data of Holter electrocardiograph of the subject recorded for 24 hours, may be transmitted to the server 20. Information input by the subject by operating the portable information terminal, such as "took a medicine", "have a headache", or the like, for example, may be transmitted to the server 20.

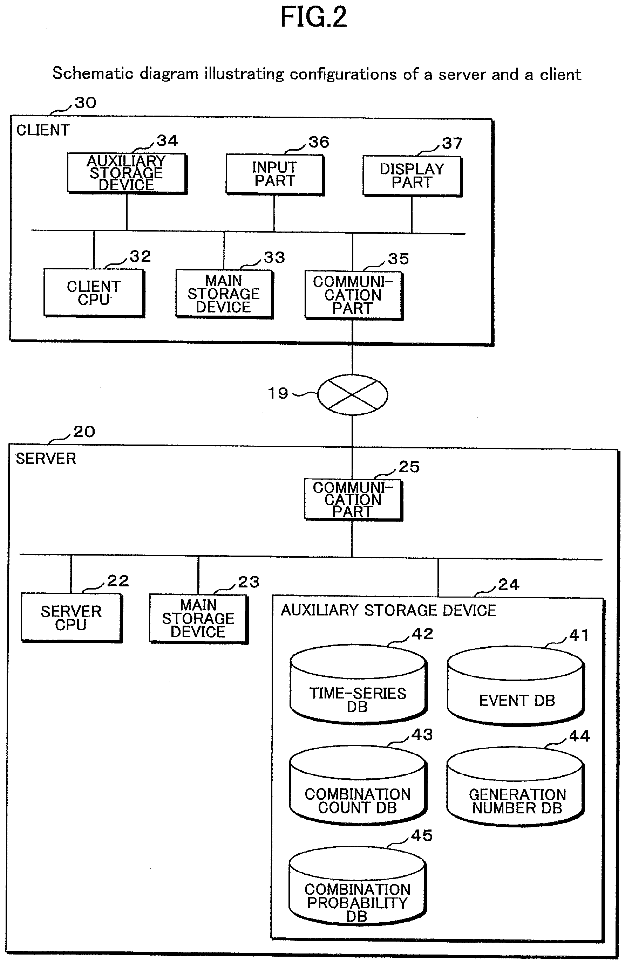

FIG. 2 is a schematic diagram illustrating configurations of the server 20 and the client 30. The server 20 includes a server CPU (Central Processing Unit) 22, a main storage device 23, an auxiliary storage device 24, a communication part 25, and a bus. The server 20 of this embodiment is a general-purpose personal computer, or a large-scale computer or the like set up at a data center.

The server CPU 22 is an ACU (Arithmetic and Control Unit) that executes a program of this embodiment. One or a plurality of CPUs, or a multi-core CPU, or the like is used for the server CPU 22. The server CPU 22 is connected to each hardware part forming the server 20, via the bus.

The main storage device 23 is a storage device such as a SRAM (Static Random Access Memory), a DRAM (Dynamic Random Access Memory), a flash memory, or the like. Information required during processes performed by the server CPU 22, and the program being executed by the server CPU 22 are temporarily stored in the main storage device 23.

The auxiliary storage device 24 is a storage device such as a SRAM, a flash memory, a hard disk, a magnetic tape, or the like. The program to be executed by the server CPU 22, and various information required to execute the program, such as an event DB (Data-Base) 41, a time-series DB 42, a combination count DB 43, a generation number DB 44, a combination probability DB 45, or the like are stored in the auxiliary storage device 24. The communication part 25 is an interface that performs a communication via the network 19.

The client 30 includes a client CPU 32, a main storage device 33, an auxiliary storage device 34, a communication part 35, an input part 36, a display part 37, and a bus. The client 30 in this embodiment is a general-purpose personal computer, a tablet, a smartphone, or the like.

The client CPU 32 is an ACU that executes a program of this embodiment. One or a plurality of CPUs, or a multi-core CPU, or the like is used for the client CPU 32. The client CPU 32 is connected to each hardware part forming the client 30, via the bus.

The main storage device 33 is a storage device such as a SRAM, a DRAM, a flash memory, or the like. Information required during processes performed by the client CPU 32, and the program being executed by the client CPU 32 are temporarily stored in the main storage device 33.

The auxiliary storage device 34 is a storage device such as a SRAM, a flash memory, a hard disk, a magnetic tape, or the like. The program to be executed by the client CPU 32, and various information required to execute the program are stored in the auxiliary storage device 34. The communication part 35 is an interface that performs a communication via the network 19.

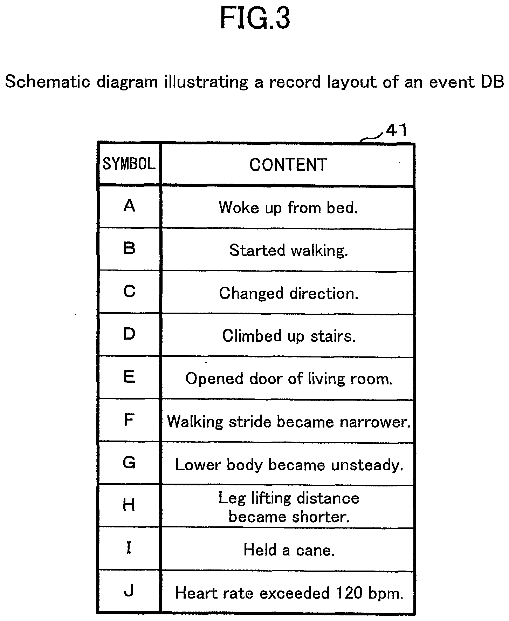

FIG. 3 is a schematic diagram illustrating a record layout of the event DB 41. The event DB 41 is a DB that relates a symbol and contents of a change in the state of the subject processed by the program of this embodiment. The event DB 41 includes a symbol field, and a content field. The symbol indicated by a character, a numerical value, or the like is recorded in the symbol field. The contents of the change in the state of the subject indicated by the symbol recorded in the symbol field is recorded in the content field. FIG. 3 illustrates examples of records recorded with operations of the subject, such as "woke up from bed", or the like, however, the records may include changes in an ambient environment of the subject, such as "illumination of bedroom turned off", "room temperature exceeded 28 degrees", or the like, for example, recorded in the content field. In addition, the records may include lapses of durations of states of the subject, such as "walking time exceeded 10 minutes", or the like, for example, recorded in the content field. In the following description, even in a case of an event indicating the lapse of the duration, the event may be described as being an event that is generated, or an event that occurred. The symbol DB 41 includes 1 record for 1 symbol.

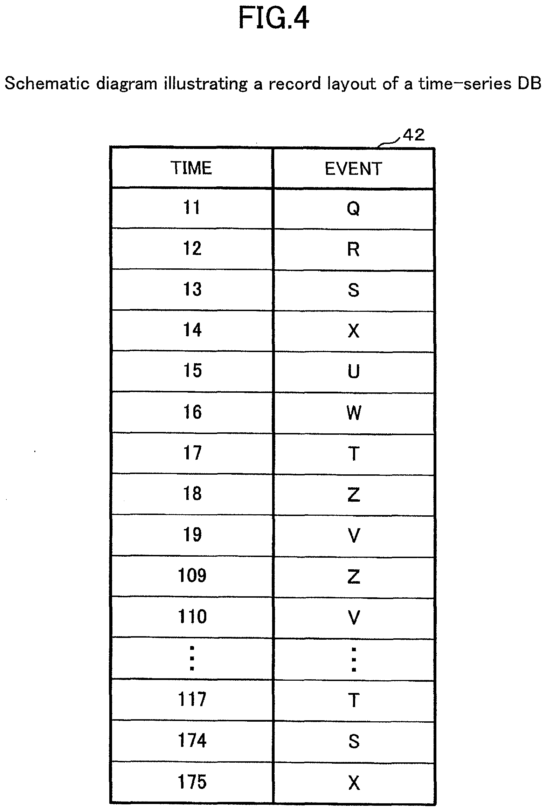

FIG. 4 is a schematic diagram illustrating a record layout of the time-series DB 42. The time-series DB 42 is a DB that relates the symbol indicating the generated event, and a time related to the event, such as a time when the event is generated, for example. The time-series DB 42 includes a time field, and an event field. The time field is recorded with a numerical value indicating a date and time. In order to simplify the description, a unit "seconds" is used in the following description for the numerical value recorded in the time field. The numerical value used in the time field identifies the time by a unit that is small to such an extent that a plurality of events are not generated simultaneously, such as microseconds, nanoseconds, or the like, for example.

The event field is recorded with the symbol indicating the change in the state of the subject generated at the time that is recorded in the time field. It is possible to know the contents of the event that is actually generated, by searching the event DB 41 using the symbol recorded in the event field as a key. The time-series DB 42 includes 1 record for 1 time corresponding to the event. The records of the time-series DB 42 are arranged in an ascending order of the time fields.

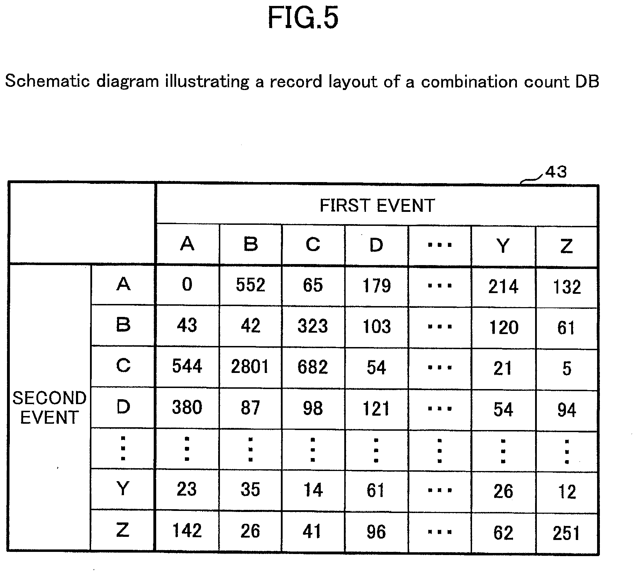

FIG. 5 is a schematic diagram illustrating a record layout of the combination count DB 43. The combination count DB 43 is a DB that records a count of second events generated within a predetermined time after a first event. The predetermined time refers to a time for which causality or correlation that the second event is generated may exist, by being affected by the generation of the first event. In the following description, this predetermined time will be referred to an affected time. A length of the affected time is 30 seconds, for example.

The combination count DB 43 includes a first event field, and a second event field. The first event field includes fields corresponding to the symbols recorded in the symbol field of the event DB 41 described in conjunction with FIG. 3. The second event field includes fields recorded with the corresponding symbols recorded in the symbol field of the event DB 41 described in conjunction with FIG. 3. The combination count DB 43 includes a number of records identical to the number of symbols recorded in the symbol field of the event DB 41. The combination count DB 43 is created by analyzing the time-series DB 42.

For example, a numerical value 179 is recorded in a D-field of the first event field, for the record having A recorded in the second event field illustrated in FIG. 5. In this case, it is indicated that, after the event of the D-field is generated, the event A is generated 179 times within the predetermined affected time. The affected time is the time in which the generated event may affect another event. The affected time is stored in advance in the auxiliary storage device 24. In this embodiment, a description is given of a case in which the affected time is 20 seconds, for example.

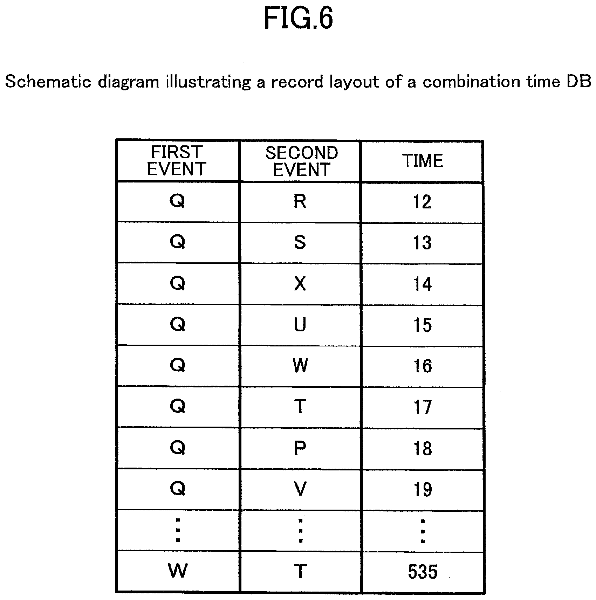

FIG. 6 is a schematic diagram illustrating a record layout of a combination time DB. The combination time DB is a DB that records a time when the second event is generated within the predetermined affected time after the first event. The combination time DB includes a first event field, a second event field, and a time field. The first event field is recorded with a symbol indicating the first event. The second event field is recorded with a symbol indicating the second event. The time field is recorded with the time when the second event is generated. The combination time DB includes 1 record for 1 combination of the first event and the second event generated within the affected time.

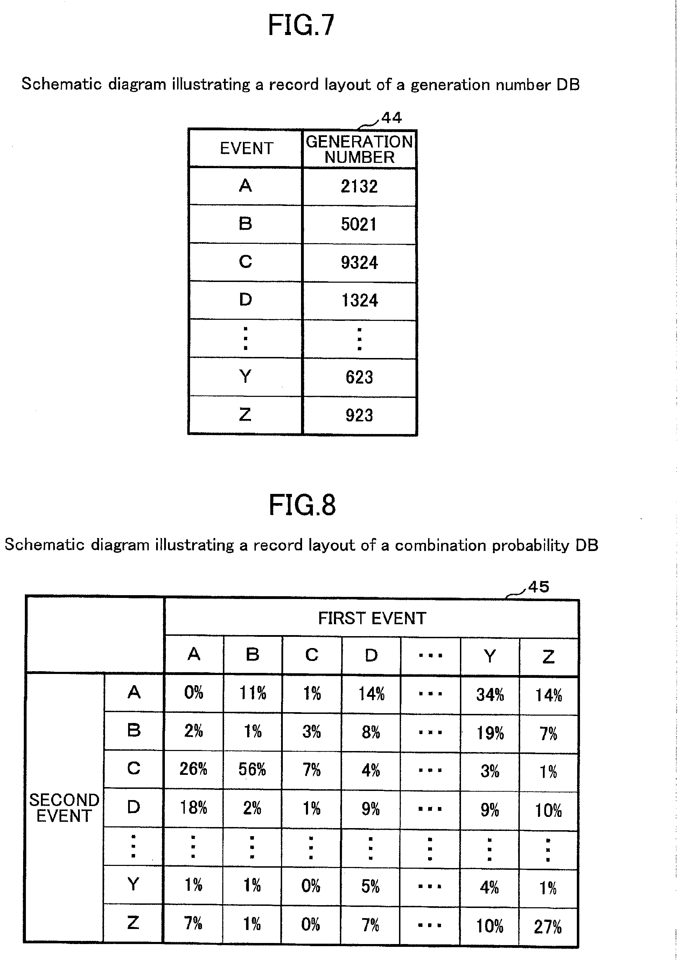

FIG. 7 is a schematic diagram illustrating a record layout of the generation number DB 44. The generation number DB 44 is a DB that relates each event and a generation number of each event. The generation number DB 44 includes an event field, and a generation number field. The event field is recorded with a symbol corresponding to the symbol field of the event DB 41 described in conjunction with FIG. 3. The generation number field is recorded with the generation number of each event. The generation number DB 44 includes a number of records identical to the number of symbols recorded in the symbol field of the event DB 41. The generation number DB 44 is created by analyzing the time-series DB 42.

FIG. 8 is a schematic diagram illustrating a record layout of the combination probability DB 45. The combination probability DB 45 is a DB that records a probability that the second event is generated within the predetermined affected time after the first event. The combination probability DB 45 includes a first event field, and a second event field. The first event field includes fields corresponding to the symbols recorded in the symbol field of the event DB 41 described in conjunction with FIG. 3. The second event field includes fields recorded with the corresponding symbols recorded in the symbol field of the event DB 41 described in conjunction with FIG. 3. The combination count DB 43 includes a number of records identical to the number of symbols recorded in the symbol field of the event DB 41. The combination count DB 43 is created by combining the combination count DB 43 and the generation number DB 44.

For example, a numerical value 14% is recorded in the C-field of the first event field, for the record having A recorded in the second event field illustrated in FIG. 8. In this case, after the event D illustrated in FIG. 5 is generated, the event A is generated 179 times within the predetermined affected time, the event D illustrated in FIG. 7 is generated 1324 times, and the numerical value represents a ratio, in percentage, of the number of times the event A is generated to the number of times the event D is generated.

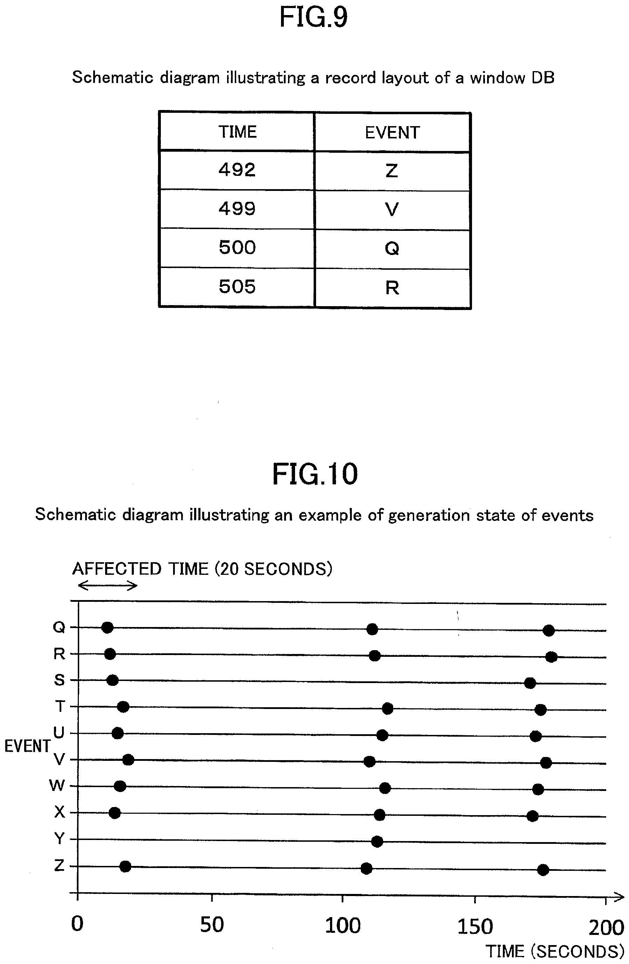

FIG. 9 is a schematic diagram illustrating a record layout of a window DB. The window DB is a DB that is obtained from the time-series DB 42 by extracting the records in which the time recorded in the time field is within a range of the affected time. The window DB includes a time field and an event field. The time field is recorded with a numerical value indicating the date and time. The time-series DB 42 includes 1 record for 1 time corresponding to the event. The window DB is successively created based on the time-series DB 42.

FIGs. 10 through 13 are schematic diagrams illustrating an example of the generation state of the event. A description will be given of an example of the event analyzed in this embodiment, by referring to FIGs. 10 through 13. In FIGs. 10 through 13, an abscissa indicates the time, and an ordinate indicates the symbol of the event. A block circle indicates that an event indicated on the ordinate is generated at the time indicated on the abscissa. FIGs. 10 through 13 illustrate information recorded in the time-series DB 42 described in conjunction with FIG. 4.

FIG. 10 indicates the generation state of the event at times in a range from 0 second to 200 seconds. In this embodiment, the events are concentrated at time bands shorter than the affected time of 20 seconds, in a vicinity of 20 seconds, in a vicinity of 110 seconds, and in a vicinity of 170 seconds. The time bands in which the respective events are concentrated are separated by more than the affected time of 20 seconds.

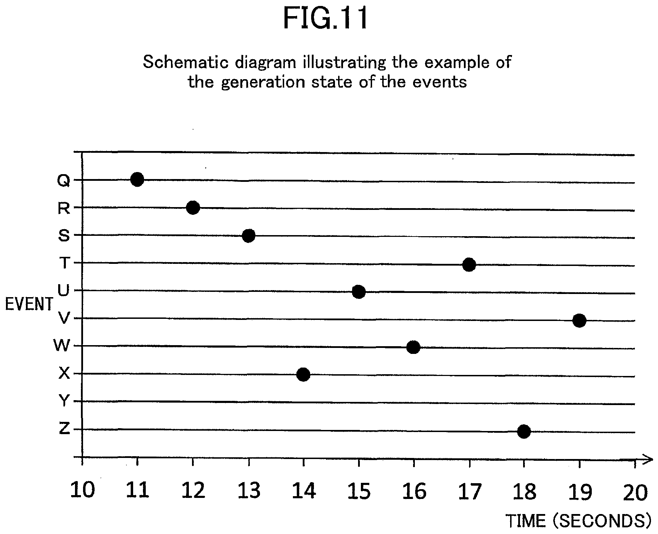

FIG. 11 is a diagram in which the abscissa in FIG. 10 is expanded in a range from 10 seconds to 20 seconds. A set of 9 events indicated by the black circles in FIG. 11 are generated within the range of the affected time, and thus, there is a possibility that some kind of causality or the correlation exists between 1 event and an event that is generated thereafter. In addition, there is a possibility that the plurality of events were generated at adjacent times simply by accident. In a case in which a large amount of data is collected and analyzed, it may be regarded that there is a high possibility of the correlation or the causality existing between the events having a high probability of being generated consecutively within the affected time. A description will hereinafter be given of a method of computing a generation probability of events generated consecutively within the affected time.

As illustrated in FIG. 10, the set of 9 events indicated by the black circles in FIG. 11 are separated in time from events other than those of the set, by the affected time or longer. Accordingly, it may be assumed that these 9 events do not affect generation of the events other than those of the set, and are unaffected by the generation of the events other than those of the set. Hence, it is possible to analyze the data in the range from 10 seconds to 20 seconds by checking a relationship of the 9 events illustrated in FIG. 11 by round-robin. In the following description, the set of events in a range shorter than the affected time and separated in time from the events other than those of the set by the affected time or longer, that is, the set of events that is isolated, will be referred to as a complete isolation set.

A specific description will be given of a method of counting the combination of events between which the causality or the correlation may exist. After an event Q generated at 11 seconds, each of events "R, S, X, U, W, T, Z, and V" is generated within the range of the affected time. Accordingly, the server CPU 22 adds 1 to records of each of the events "R, S, X, U, W, T, Z, and V" of the second event field for the Q-field of the first event field in the combination count DB 43 illustrated in FIG. 5. Similarly, after an event R is generated at 12 seconds, each of events "S, X, U, W, T, Z, and V" is generated within the range of the affected time. Accordingly, the server CPU 22 adds 1 to records of each of the events "S, X, U, W, T, Z, and V" of the second event field for the R-field of the first event field in the combination count DB 43 illustrated in FIG. 5. Hence, the server CPU 22 repeats sequentially extracting 2 events in time series from the complete isolated set, counting occurrences of those 2 events, and recording the counts in the combination count DB 43, for all complete isolated sets.

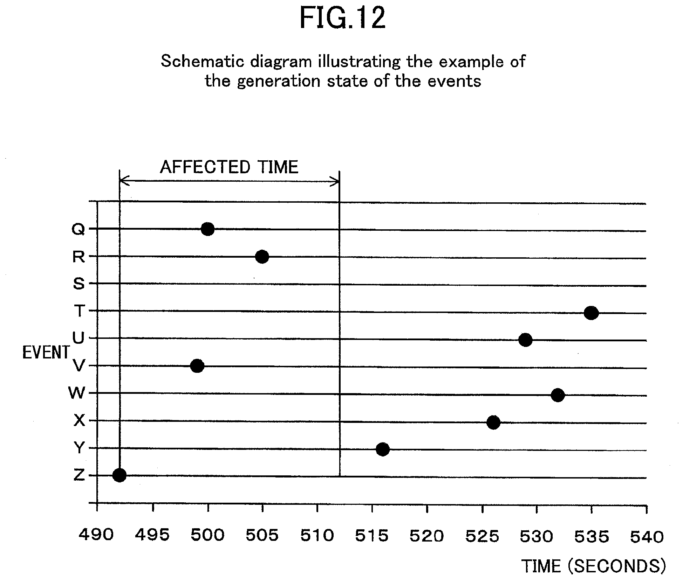

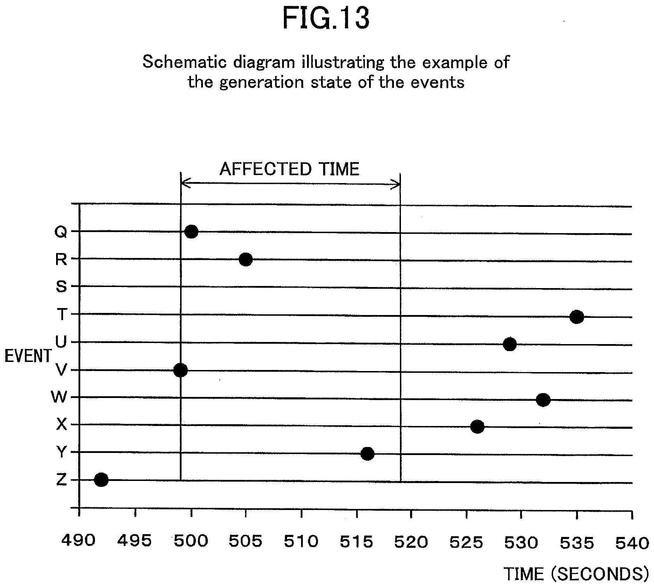

Next, a description will be given of a case in which a separation of a set from the events other than those of the set is the affected time or longer, that is, the set is isolated from the events other than those of the set, but a time from a head event to a last event within the set is longer than the affected time. In the following description, such a set will be referred to as a semi-isolation set. FIGs. 12 and 13 illustrate an example of the semi-isolation set. It is also assumed in a case of the semi-isolation set that events of the semi-isolation set do not affect generation of events other than those of the semi-isolation set, and are unaffected by the generation of the events other than those of the semi-isolation set. Further, the event Z generated at 492 seconds and the event Y generated at 526 seconds, for example, are separated by a time longer than the affected time of 20 seconds, and it may be assumed that there is no causality nor correlation between the two events.

A description will be given of a method of recording a combination count of the first event and the second event in the combination count DB 43, in the case of such a semi-isolation matrix. First, the server CPU 22 creates the window DB by extracting the events within the affected time from a 1st event, as illustrated in FIG. 12. FIG. 9 illustrates an example of the created window DB. The server CPU 22 adds 1 to records of each of the events "V, Q, and R" of the second event field within the window DB for the Z-field of the first event field within the window DB in the combination count DB 43.

Thereafter, the server CPU 22 creates the window DB by extracting the events within the affected time from a 2nd event, as illustrated in FIG. 13. The server CPU 22 adds 1 to records of each of the events "Q, R, and Y" of the second event field within the window DB for the U-field of the first event field within the window DB in the combination count DB 43. By successively shifting, on a time base, the position where the window DB is created, the combination having the possibility of having the causality or the correlation within the semi-isolation set is recorded in the combination count DB 43. The server CPU 22 repeats the above described operation for all semi-isolation matrixes.

By the operation described above, the server CPU 22 can record, in the combination count DB 43, all of the combinations of the events within the affected time, among the events recorded in the time-series DB 42.

Further, the server CPU 22 counts the number of each of the events recorded in the time-series DB 42, and records the number in the generation number DB 44. The generation number DB 44 illustrated in FIG. 7 illustrates that the number of times the event A is recorded in the time-series DB 42 is 2132 times, and the number of times the event B is recorded in the time-series DB 42 is 5021 times.

The server CPU 22 obtains a ratio of the value recorded in the A-field of each record of the combination count DB 43 to the value recorded in the generation number field of the record for the event A of the generation number DB 44, and records the ratio in the corresponding field of the combination probability DB 45. The server CPU 22 similarly obtains the ratio for the B-field and subsequent fields, to create the combination probability DB 45. The combination probability DB 45 is recorded with the probability that the second event is generated within the affected time, in the case in which the first event is generated.

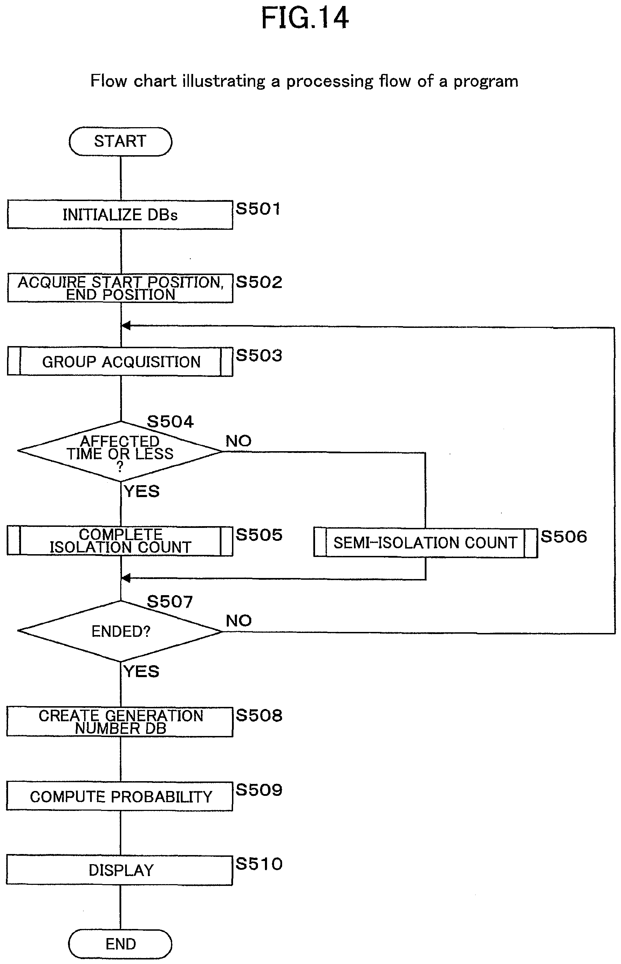

FIG. 14 is a flow chart illustrating a processing flow of a program. The program illustrated in FIG. 14 is a program for acquiring the record from the time-series DB 42, and computing the probability of the combination of 2 events being generated within the affected time. The processing flow of the program in this embodiment will be described, by referring to FIG. 14.

The server CPU 22 initializes the combination count DB 43 and the combination time DB (step S501). That is, the server CPU 22 sets the value of each of the fields of the combination count DB 43 to an initial value, which is 0. Further, the server CPU 22 deletes all of the records in the combination time DB.

The server CPU 22 acquires a start position and an end position of the analysis within the time-series DB 42 (step S502). The server CPU 22 acquires the start position and the end position of the analysis, from an input made from the input part 36 by a user such as a physician or the like who analyzes the state of the subject, via the communication part 25, the network 19, and the communication part 35. The server 22 boots a subroutine for group acquisition (step S503). The subroutine for acquiring the group of events by using the 2 temporally successive events whose time difference is longer than the affected time, from the data within the time-series DB 42. A processing flow of the subroutine for group acquisition will be described later.

The server CPU 22 judges whether the time between the head event and the last event of the acquired group is the affected time or shorter (step S504). In a case in which the time is the affected time or shorter (YES in step S504), the server CPU 22 boots a subroutine for complete isolation count (step S505). The subroutine for complete isolation count is a subroutine for recording the combination of events within the complete isolation set in the combination count DB 43. A processing flow of the subroutine for complete isolation count will be described later.

In a case in which the time is longer than the affected time (NO in step S504), the server CPU 22 boots a subroutine for semi-isolation count (step S506). The subroutine for semi-isolation count is a subroutine for recording the combination of events within the semi-isolation set in the combination count DB 43. A processing flow of the subroutine for semi-isolation count will be described later.

After step S505 or step S506, the server CPU 22 judges whether analysis of the time-series DB 42 ended (step S507). The server CPU 22 acquires the end position of the analysis in step S502, as described above. In a case in which it is judged that the analysis has not ended (NO in step S507), the server CPU 22 returns the process to step S503. In a case in which it is judged that the analysis has ended (YES in step S507), the server CPU 22 creates the generation number DB 44 by counting the number of each of the events between the start time and the end time acquired in step S502 (step S508). The server CPU 22 computes the probability that the second event is generated within the affected time in a case in which the first event is generated, from the combination count DB 43 and the generation number DB 44, and records the probability in the combination probability DB 45 (step S509). The server CPU 22 displays the combination probability DB or the like created by the processes described above, on the display part 37 of the client 30, via the communication part 25, the network 19, and the communication part 35 (step S510). An example of the display will be described later. Thereafter, the server CPU 22 ends the process.

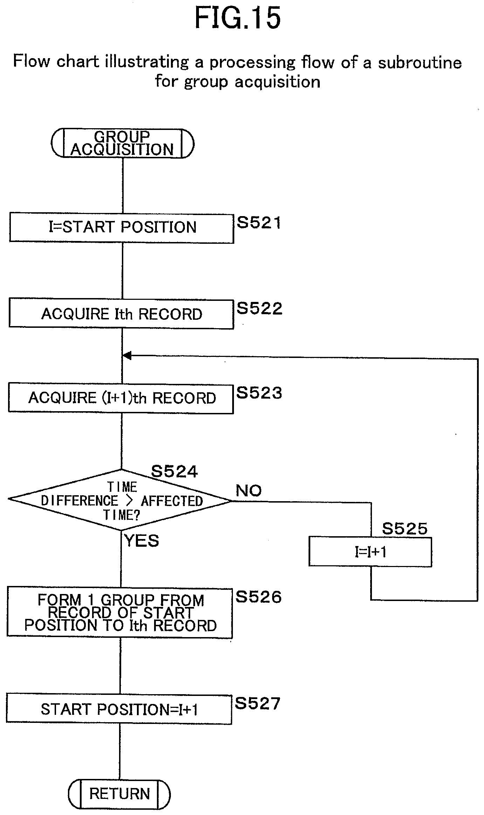

FIG. 15 is a flow chart illustrating a processing flow of the subroutine for group acquisition. The subroutine for group acquisition is the subroutine for acquiring the group of events each separated by the affected time or longer from 1 preceding event and from 1 subsequent event, from the data within the time-series DB 42.

The server CPU 22 sets a counter I to the start position (step S521). The start position is a number indicating the first record that starts the process of the subroutine for group acquisition within the time-series DB 42. In a case in which this subroutine is executed for the first time, the start position is the number of the record within the time-series DB 42, corresponding to a time when the analysis of the accepted input from the user starts. In a case in which this subroutine is executed for the second or subsequent times, the start position is the value recorded in the auxiliary storage device 24 at a time when execution of this subroutine executed the previous time ended.

The server CPU 22 acquires an Ith record from the time-series DB 42 (step S522). The server CPU 22 acquires a (I+1)th record from the time-series DB 42 (step S523). The server CPU 22 judges whether a time difference between the (I+1)th record and the Ith record is greater than the affected time (step S524). In a case in which it is judged that the time difference is the affected time or less (NO in step S524), the server CPU 22 adds 1 to the counter I (step S525). Thereafter, the server CPU 22 returns the process to step S523).

In a case in which it is judged that the time difference is greater than the affected time (YES in step S524), the server CPU 22 determines the record corresponding to the start position used in step S521 up to the Ith record, as belonging to 1 group (step S526). The server CPU 22 records the start position as being I+1 (step S527). The server CPU 22 thereafter ends the process.

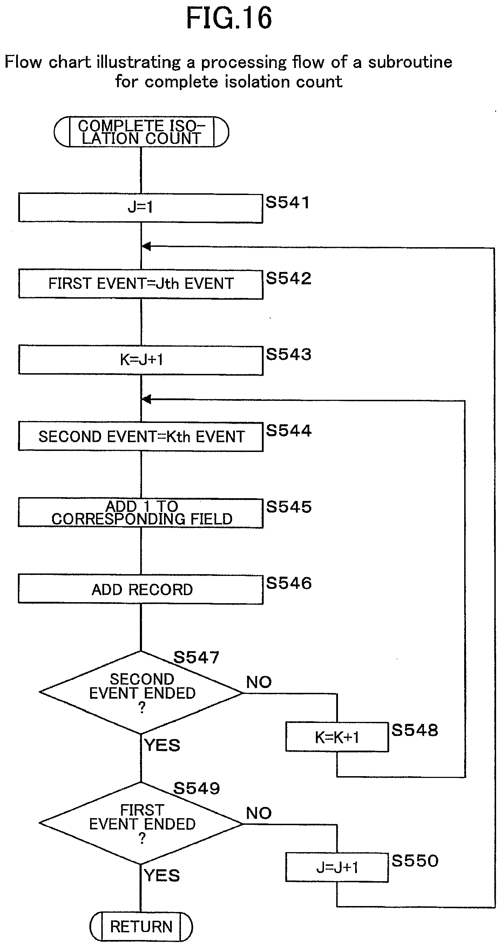

FIG. 16 is a flow chart illustrating a processing flow of the subroutine for complete isolation count. The subroutine for complete isolation count is the subroutine for recording the combination of the events within the complete isolation set in the combination count DB 43.

The server CPU 22 sets a counter J to an initial value, which is 1 (step S541). The server CPU 22 sets a Jth event within the group to the first event (step S542). The server CPU 22 sets a counter K to J+1 (step S543). The server CPU 22 sets a Kth event within the group to the second event (step S544). The server CPU 22 adds 1 to a corresponding field of the combination count DB 43, that is, the field of the first event in the record of the second event (step S545). The server CPU 22 adds a record recorded with the first event, the second event, and the time related to the second event, in the combination time DB (step S546).

The server CPU 22 judges whether the second event ended (step S547). In a case in which the last event within the group is the second event, the server CPU 22 judges that the second event ended. In a case in which it is judged that the second event has not ended (NO in step S547), the server CPU 22 adds 1 to the counter K (step S548). Thereafter, the server CPU 22 returns the process to step S544.

In a case in which it is judged that the second event ended (YES in step S547), the server CPU 22 judges whether the first event ended (step S549). In a case in which the second to last event within the group is the first event, the server CPU 22 judges that the first event ended. In a case in which it is judged that the first event has not ended (NO in step S549), the server CPU 22 adds 1 to the counter J (step S550). Thereafter, the server CPU 22 returns the process to step S542. In a case in which it is judged that the first event ended (YES in step S549), the server CPU 22 ends the process.

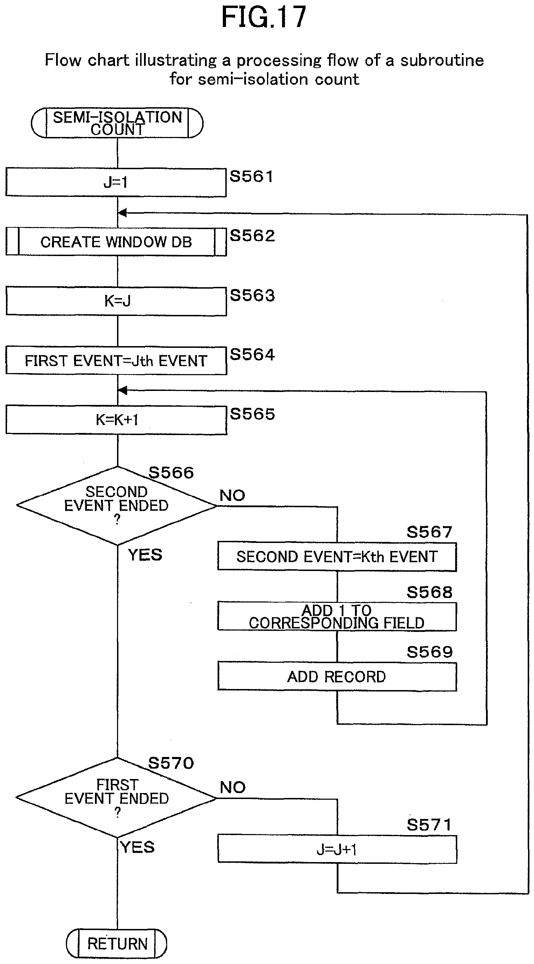

FIG. 17 is a flow chart illustrating a processing flow of the subroutine for semi-isolation count. The subroutine for semi-insolation count is the subroutine for recording the combination of events within the semi-isolation set in the combination count DB 43.

The server CPU 22 sets the counter J to an initial value, which is 1 (step S561). The server CPU 22 boots a subroutine for creating the window DB (step S562). The subroutine for creating the window DB is a subroutine for creating the window DB from the time-series DB 42 by extracting the records in which the time recorded in the time field is within the range of the affected time. A processing flow of the subroutine for creating the window DB will be described later.

The server CPU 22 sets the counter K to the same value as the counter J (step S563). The server CPU 22 sets the Jth event within the group to the first event (step S564). The server CPU 22 sets the counter K to K+1 (step S565). The server CPU 22 judges whether the second event ended (step S566). In a case in which the step Kth event does not exist within the window DB, the server CPU 22 judges that the second event ended.

In a case in which it is judged that the second event has not ended (NO in step S566), the server CPU 22 sets the Kth event within the window DB to the second event (step S567). The server CPU 22 adds 1 to a corresponding field of the combination count DB 43, that is, the field of the first event in the record of the second event (step S568). The server CPU 22 adds a record recorded with the first event, the second event, and the time related to the second event, in the combination time DB (step S569). Thereafter, the server CPU 22 returns the process to step S565.

In a case in which it is judged that the second event has ended (YES in step S566), the server CPU 22 judges whether the first event ended (step S570). In a case in which the second to last event within the group is the first event, the server CPU 22 judges that the first event ended. In a case in which it is judged that the first event has not ended (NO in step S570), the server CPU 22 adds 1 to the counter J (step S571). Thereafter, the server CPU 22 returns the process to step S562. In a case in which it is judged that the first event ended (YES in step S570), the server CPU 22 ends the process.

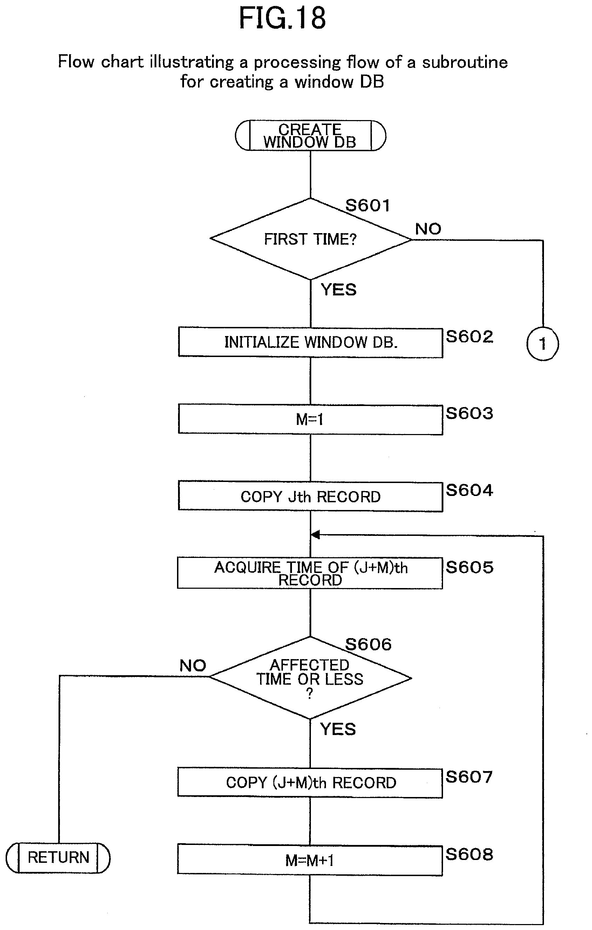

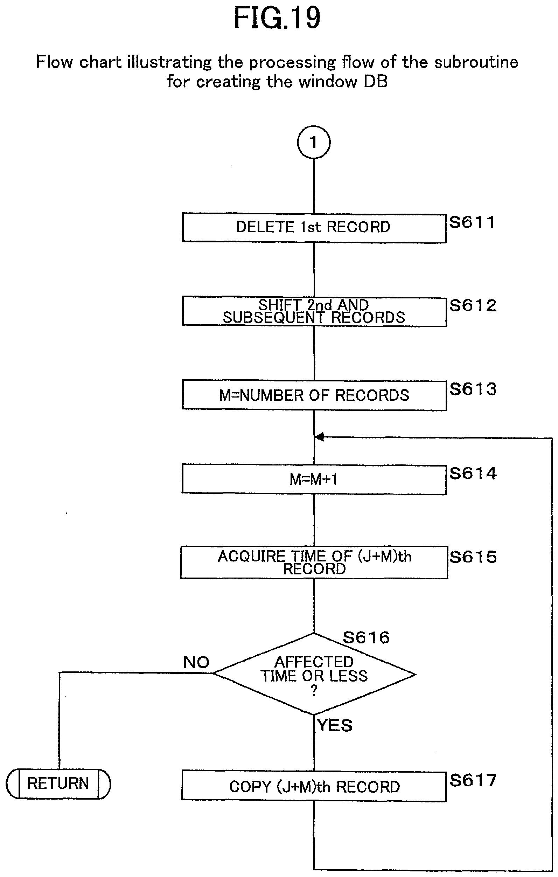

FIGs. 18 and 19 are flow charts illustrating a process flow of the subroutine for creating the window DB. The subroutine for creating the window DB is a subroutine for creating the window DB from the time-series DB 42 by extracting the records in which the time recorded in the time field is within the range of the affected time.

The server CPU 22 judges whether the subroutine is executed for the first time (step S601). In a case in which the subroutine is executed for the first time (YES in step S601), the server CPU 22 initializes the window DB by detecting all records of the window DB (step S602). The server CPU 22 sets a counter M to 1 (step S603). The server CPU 22 copies the Jth record of the time-series DB 42 to the window DB (step S604). In this case, the counter J indicates the number of the record from which the creation of the window DB starts, set by the subroutine at a calling source.

The server CPU 22 acquires a time recorded in the time field of a (J+M)th record from the time-series DB 42 (step S605). The server CPU 22 judges whether a time between the Jth record and the (J+M)th record is the affected time or less (step S606). In a case in which it is judged that the time between the Jth record and the (J+M)th record is the affected time or less (YES in step S606), the server CPU 22 copies the (J+M)th record of the time-series DB 42 to the window DB (step S607). The server CPU 22 adds 1 to the counter M (step S608). The server CPU 22 returns to the process to step S605. In a case in which it is judged that the time between the Jth record and the (J+M)th record exceeds the affected time (NO in step S606), the server CPU 22 ends the process.

In a case in which the subroutine is executed for the second or subsequent times (NO in step S601), the server CPU 22 deletes the 1st record from the window DB (step S611). The server CPU 22 shifts the records one by one, so that the second record is shifted to the first record, the third record is shifted to the second record, and so on (step S612). The shift operation is performed by changing the address of the memory storing the data within the auxiliary storage device 24 or the main storage device 23.

The server CPU 22 sets the counter M to the number of records within the window DB (step S613). The server CPU 22 adds 1 to the counter M (step S614). The server CPU 22 acquires a time recorded in the time field of the (J+M)th record from the time-series DB 42 (step S615). The server CPU 22 judges whether the time between the Jth record and the (J+M)th record is the affected time or less (step S616). In a case in which it is judged that the time between the Jth record and the (J+M)th record is the affected time or less (YES in step S616), the server CPU 22 copies the (J+M)th record of the time-series DB 42 to the window DB (step S617). The server CPU 22 returns the process to step S614. In a case in which it is judged that the time between the Jth record and the (J+M)th record exceeds the affected time (NO in step S616), the server CPU 22 ends the process.

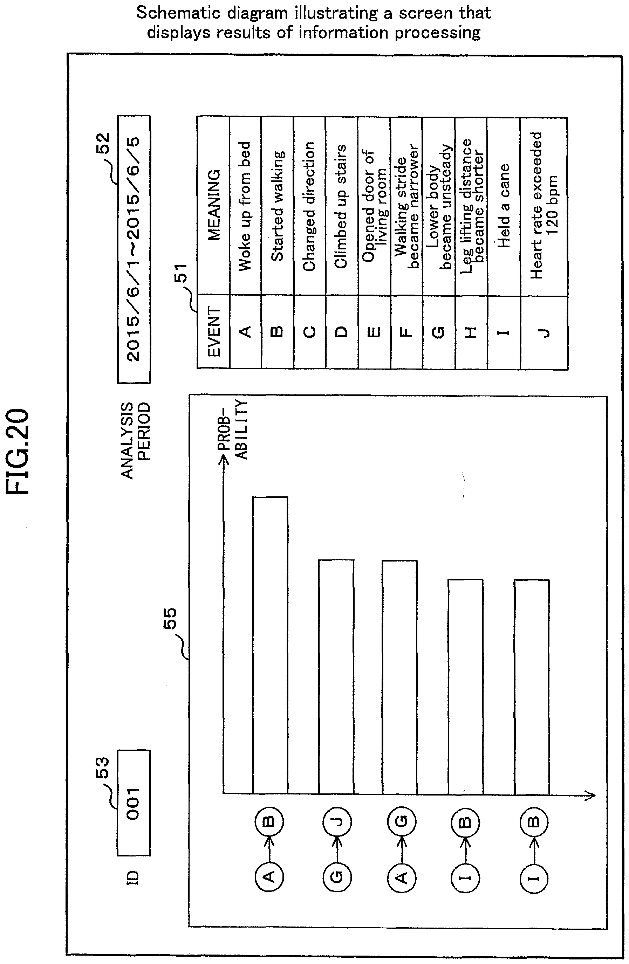

FIGs. 20 through 22 are schematic diagrams illustrating screens that display results of information processing. FIGs. 20 through 22 are examples of the screens that display information recorded in the combination probability DB 45 or the combination time DB on the display part 37 of the client 30, according to an accepted operation from the user made from the input part 36. Various existing techniques may be used for procedures of extracting data and generating graphs from the combination probability DB 45 or the combination time DB, and thus, a description on such techniques will be omitted.

FIG. 20 is an example of the screen that displays high ranked items extracted from the combination probability DB 45. The screen displays a list field 51, an analysis period field 52, an ID (Identification) field 53, and a data field 55. The list field 51 displays contents of the record recorded in the event DB 41. In a case in which not all of the records recorded in the display DB 41 are displayable on the display part 37, it is desirable to extract the records corresponding to the information displayed in the data field 55 to be described later, and display the extracted records in the list field 51.

The analysis period field 52 is a field for accepting input of the range of the data to be extracted from the time-series DB 42 and analyzed. The client CPU 32 accepts the input to the analysis period field 52 via the input part 36, and transmits the accepted input to the server CPU 22 via the communication part 35, the network 19, and the communication part 25.

The ID field 53 is a field for accepting input of an ID peculiar to the subject whose data is to be analyzed. In a case in which the data of a plurality of subjects are recorded in the auxiliary storage device 24, the data of each of the individual subjects is distinguished by the ID. The ID is recorded in a field that records a file name of each DB or an ID provided for each DB. The client CPU 32 accepts the input to the ID field 53 via the input part 36, and transmits the accepted input to the server CPU 22 via the communication part 35, the network 19, and the communication part 25. In a case in which the information processing system 10 in this embodiment processes only the data of a particular subject, the ID field 53 may be omitted.

The data field 55 is a graph that visualizes the combination of the first event and the second event of the high ranked data, by the value of each of the fields within the combination probability DB 45. An abscissa of the data field 55 indicates the probability that the second event is generated when the first event is generated. An ordinate of the data field 55 indicates the combination of the first event and the second event. For example, a representation A→B indicates the probability that the second event B is generated within the affected time when the first event A is generated. The user can easily recognize, from the screen of FIG. 20, which combination of events has a high generation probability.

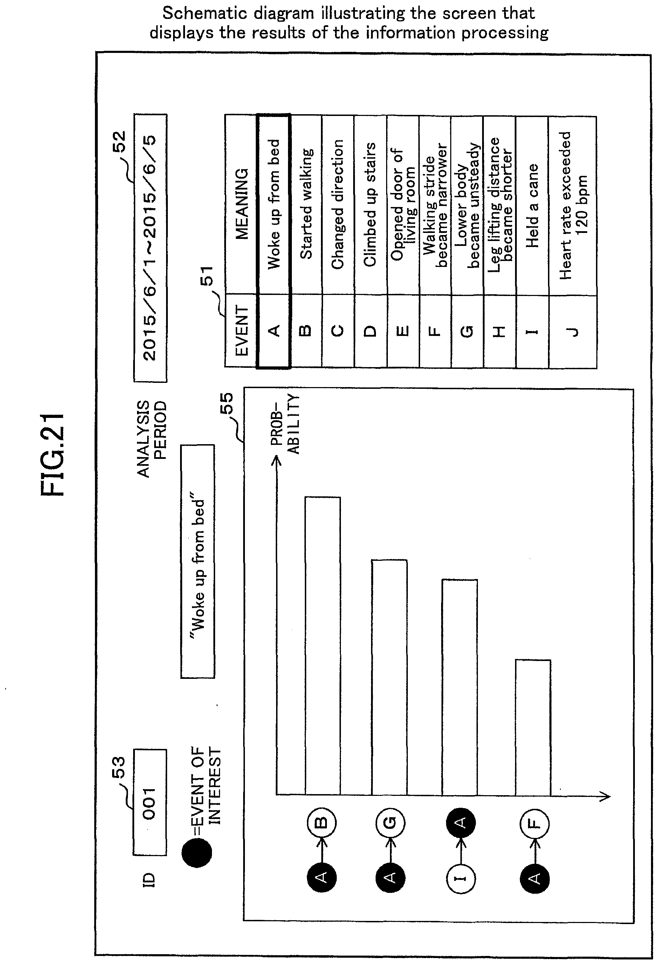

FIG. 21 illustrates the screen for a case in which a selection of the event A within the list field 51 of FIG. 20 is accepted. FIG. 21 is an example of the screen that displays high ranked items related to a predetermined event extracted from the combination probability DB 45. The data field 55 displays, in a descending order, the values in the A-fields of the first event or the second event from the combination probability DB 45. The abscissa of the data field 55 indicates the probability that the second event is generated when the first event is generated. The ordinate of the data field 55 indicates the combination of the first event and the second event. The user can easily recognize, from the screen of FIG. 21, which event has a high probability of being generated in combination with a specific event.

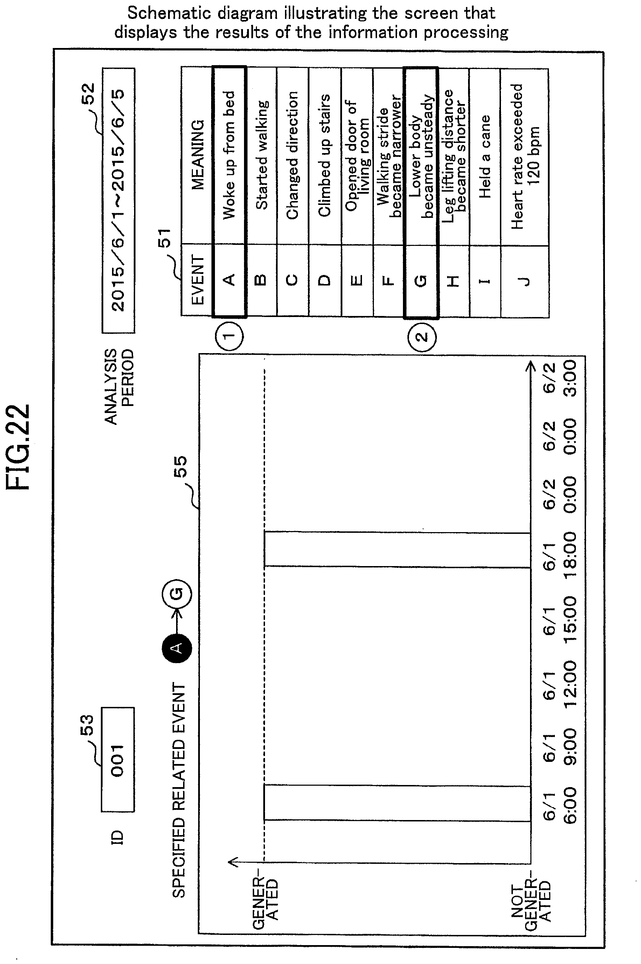

FIG. 22 illustrates the screen for a case in which selection of the event A and the event G within the list field 51 are accepted in an order. FIG. 22 is a graph that visualizes a time-series of generated times, by extracting records of combinations of predetermined first and second events from the records recorded in the combination time DB. The data field 55 displays the times when the combination of the first event A and the second event G is generated. The abscissa of the data field 55 indicates the time. The ordinate of the data field 55 indicates generation or non-generation of the events in a specified order. The user can easily recognize, from the screen of FIG. 22, the times when the combination of particular events are generated.

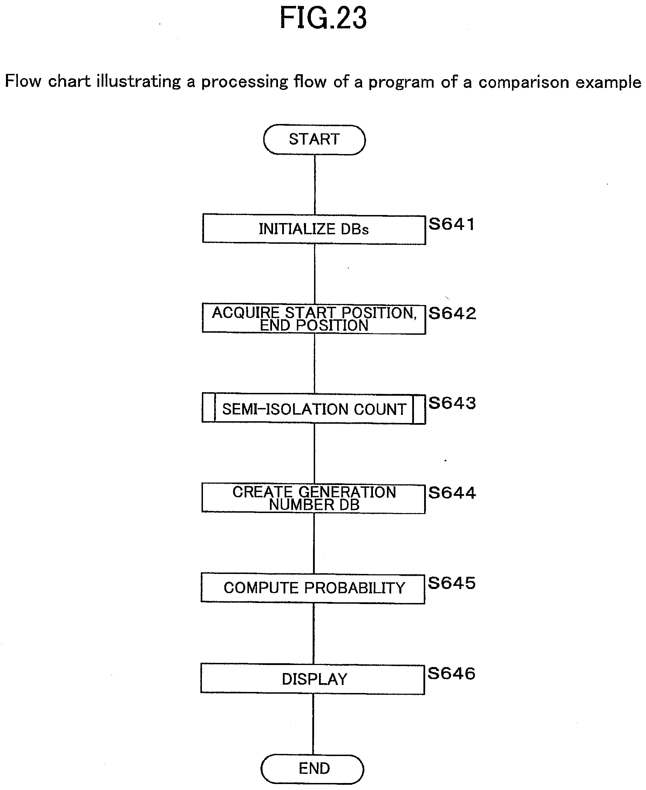

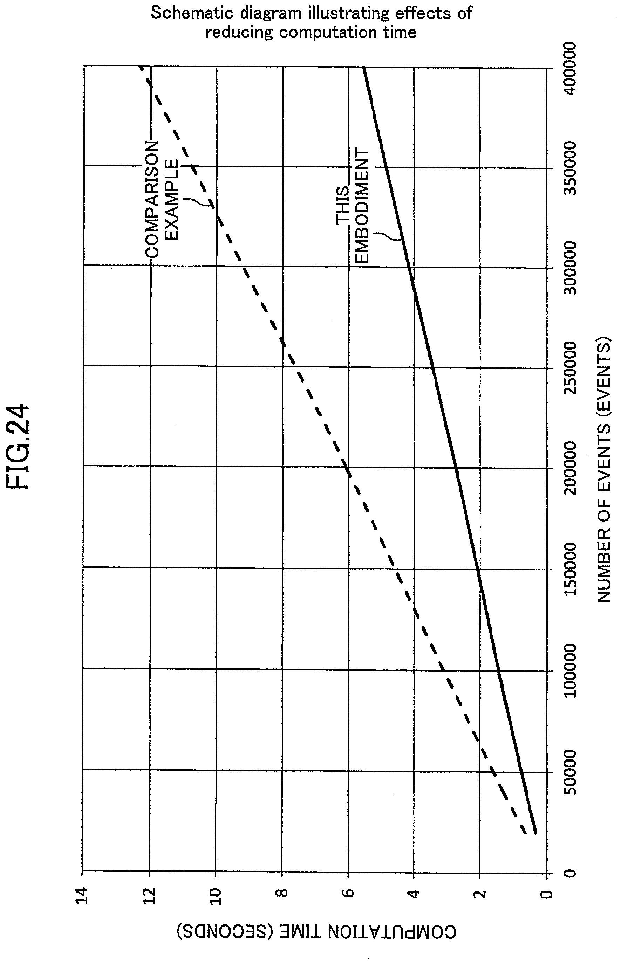

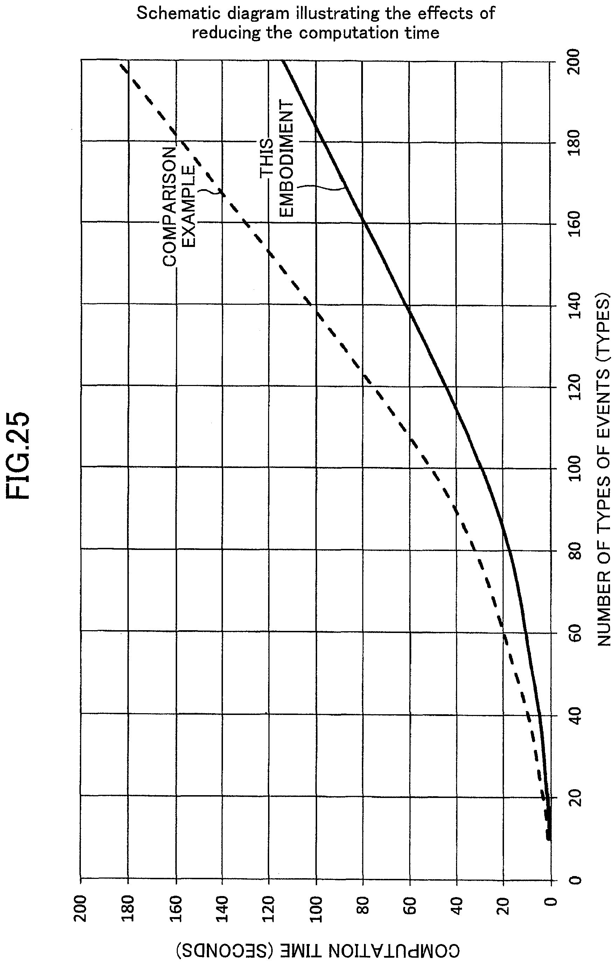

FIG. 23 is a flow chart illustrating a processing flow of a program of a comparison example. FIGs. 24 and 25 are schematic diagrams illustrating effects of reducing computation time. The effects of reducing the computation time in this embodiment will be described, by referring to FIGs. 23 through 25.

The server CPU 22 initializes the combination count DB 43 and the combination DB (step S641). In other words, the value of each field of the combination count DB 43 is set to an initial value, which is 0. In addition, all of the records of the combination time DB are deleted. The server CPU 22 acquires the start position and the end position of the analysis within the time-series DB 42 (step S642). The server CPU 22 acquires the start position and the end position of the analysis, from the input made from the input part 36 by the user such as the physician or the like who analyzes the state of the subject, via the communication part 25, the network 19, and the communication part 35.

The server CPU 22 boots a subroutine for semi-isolation count (step S643). The subroutine for semi-isolation count is a subroutine for recording the combination of the events within the semi-isolation set in the combination count DB 43. The subroutine for semi-isolation count uses the same subroutine as the subroutine described above in conjunction with FIG. 17. The subroutine for semi-isolation count processes all records of the time-series DB 42, from the start position to the end position acquired in step S632.

The server CPU 22 creates the generation number DB 44 by counting each of the events between the start time and the end time acquired in step S642 (step S644). The server CPU 22 computes the probability that the second event is generated in the case in which the first event is generated, from the combination count DB 43 and the generation number DB 44, and records the computed probability in the combination probability DB 45 (step S645). The server CPU 22 displays the combination probability DB or the like created by the processes described above, on the display part 37 of the client 30, via the communication part 25, the network 19, and the communication part 35 (step S646). For example, the screens illustrated in FIGs. 20 through 22 are examples of the display that is made.

FIG. 24 is a graph illustrating a difference in computation times between the case in which the flow chart of this embodiment illustrated in FIG. 14 is used and the case in which the flow chart of the comparison example illustrated in FIG. 23 is used. An abscissa of FIG. 24 indicates the number of records included in the time-series DB 42, that is, the number of events that are processed, and the unit of the number is events. An ordinate of FIG. 24 indicates the computation time required by the server CPU 22 in the case in which the flow chart illustrated in FIG. 24 or FIG. 14 is executed, and the unit of the computation time is seconds. The computation is made for a case in which the number of events recorded in the event DB 41 is 20 events, and the generation state of the all of the events can be divided into complete isolation sets, as illustrated in FIG. 10.

A solid line indicates the processing time for the case in which the process of this embodiment illustrated in FIG. 14 is performed, and a dotted line indicates the processing time for the case in which the process of the comparison example illustrated in FIG. 24 is performed. The processing time of this embodiment is less than or equal to one-half the processing time of the comparison example.

FIG. 25 is a graph illustrating the difference in the computation times between the case in which the flow chart of this embodiment illustrated in FIG. 14 is used and the case in which the flow chart of the comparison example illustrated in FIG. 23 is used. An abscissa of FIG. 25 indicates the number of records included in the time-series DB 42, that is, the number of types of events that are processed, and the unit of the number of types is types. An ordinate of FIG. 25 indicates the computation time required by the server CPU 22 in the case in which the flow chart illustrated in FIG. 24 or FIG. 14 is executed, and the unit of the computation time is seconds. The computation is made for a case in which the number of events recorded in the time-series DB 42 is 100,000 events, and the generation state of the all of the events can be divided into complete isolation sets, as illustrated in FIG. 10.

A solid line indicates the processing time for the case in which the process of this embodiment illustrated in FIG. 14 is performed, and a dotted line indicates the processing time for the case in which the process of the comparison example illustrated in FIG. 24 is performed. The processing time of this embodiment is approximately one-half the processing time of the comparison example in a case in which the number of types of events is approximately 60, and is approximately 60% of the processing time of the comparison example in a case in which the number of types of events is approximately 200.

It may be seen from FIGs. 24 and 25 that the computation time can be greatly reduced in this embodiment, by processing the events recorded in the time-series DB 42 after dividing the events into the groups separated by the affected time or longer as in this embodiment.

A description will be given of the reason the computation time can be reduced. In the comparison example, the entire record in the processing range within the time-series DB 42 is treated as a single semi-isolation set, and the combination of events within the affected time is extracted using the window DB. When updating the window DB, an operation is performed to shift the records within the window DB, one by one, as illustrated in step S612 of FIG. 19. This operation is an operation of changing the recorded positions of the data recorded in the main storage device 23 or the auxiliary storage device 24, and thus requires time.

On the other hand, this embodiment divides the records in the processing range within the time-series DB 42 into the complete isolation set and the incomplete isolation set. In the case of the complete isolation set, no process using the window DB is generated, and the computation time can be reduced. In addition, the computation time can also be reduced in the case of the incomplete isolation set, because the operation of shifting the records in the window DB can be reduced by an amount corresponding to processes spanning the sets, that are unnecessary.

According to this embodiment, it is possible to provide a program or the like that can efficiently extract the combination of related events, from the time-series data recorded with the generation times of a large number of events.

In a case in which the generation time of the combination of events illustrated in FIG. 22 is not displayed, it is possible to omit the process of creating the combination time DB.

It is possible to extract the generation probability of the combination of 3 or more events, by providing fields for third and subsequent events in the combination count DB 43 and the combination probability DB 45.

An analyzing target is not limited to behavior of a single subject. For example, by recording behaviors of a plurality of consumers within a shop in the time-series DB 42, it is possible to analyze combinations of the behaviors by the technique of this embodiment. In addition, the analyzing target is not limited to the behavior of the subject in a real world. For example, by recording the behavior of the subject on a network, such as web-browsing, internet-shopping, or the like in the time-series DB 42, it is possible to analyze the combination of the behaviors using the technique of this embodiment.

The combination probability DB 45 may be copied to the auxiliary storage device 34 of the client 30, and visualized by a graph or the like using general-purpose software, such as spreadsheet software or the like installed in the client 30, for example. In this case, it is possible to omit displaying the data in step S510 during the process of the program described in conjunction with FIG. 14.

In step S509, the server CPU 22 may compute the probability that the first event is generated in the case in which the second event is generated, and record the computed probability in the combination probability DB 45. In addition, two combination probability DBs 45 may be created, namely, one combination probability DB 45 for a case in which the computation is made using the first event as a reference, and another combination probability DB 45 for a case in which the computation is made using the second event as the reference. In this case, it is desirable to accept an input from the user on which one of the two combination probability DBs 45 is to be used to display the data.

### [Embodiment 2]

This embodiment relates to an information processing apparatus or the like that performs division into groups when recording the events in the time-series DB 42. A description of the parts in common with the embodiment 1 will be omitted.

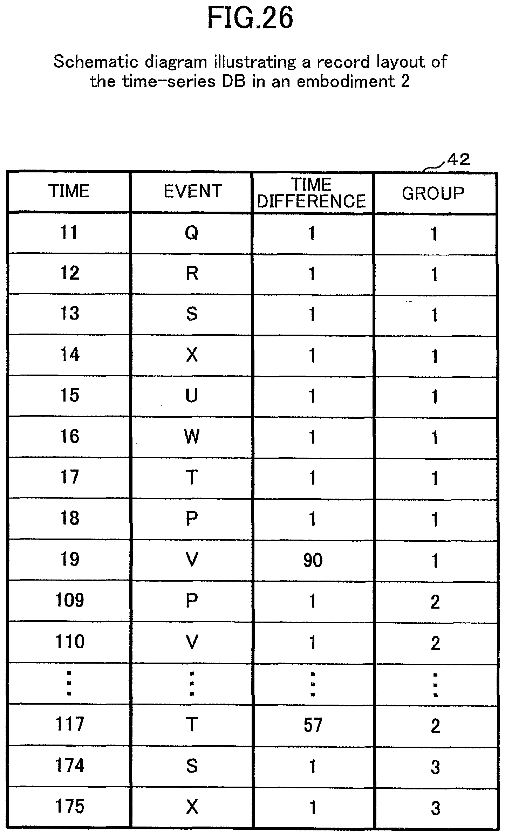

FIG. 26 is a schematic diagram illustrating a record layout of the time-series DB 42 in the embodiment 2. The time-series DB 42 in this embodiment is a DB that relates the symbol indicating the generated event, the time related to the event, and a group number. The time-series DB 42 includes a time field, an event field, a time difference field, and a group field. The time field is recorded with a numerical value indicating a date and time.

The event field is recorded with the symbol indicating the change in state of the subject at the time recorded in the time field. The time difference field is recorded with a time difference between an event and a next event. The group field is recorded with the group number of the group to which the record belongs. The time-series DB 42 includes 1 record for 1 time corresponding to the event. The records of the time-series DB 42 are arranged in an ascending order of the time fields.

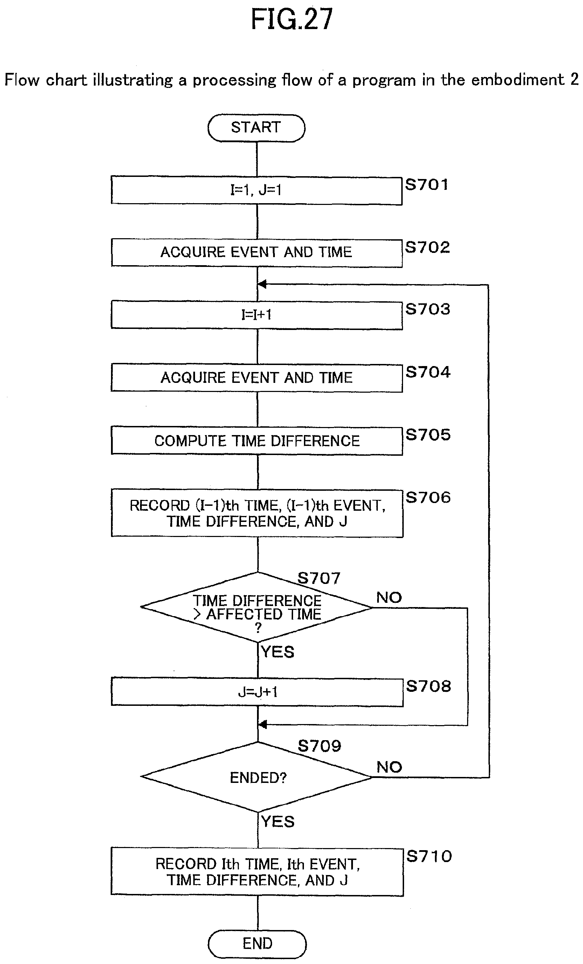

FIG. 27 is a flow chart illustrating a processing flow of a program in the embodiment 2. FIG. 27 illustrates the processing flow of the program that is executed by the server CPU 22 when recording the data in the time-series DB 42.

The server CPU 22 sets the counter I and the counter J to an initial value, which is 1 (step S701). The counter I is a counter that records acquisition orders of the event and the time. In addition, the counter J is a counter that records the group number.

The server CPU 22 acquires the event and time from the sensor control device 12, the biometric sensor 16, or the like, via the network 19 (step S702). The server CPU 22 adds 1 to the counter I (step S703). The server CPU 22 acquires the next event and time from the sensor control device 12, the biometric sensor 16, or the like, via the network 19 (step S704). The server CPU 22 computes a time difference between the time acquired in step S702 and the time acquired in step S704 (step S705).

The server CPU 22 creates a record recorded with a (I-1)th acquired time, a (I-1)th acquired event, the time difference computed in step S705, and the counter J, and records the record in the time-series DB 42 (step S706). The server CPU 22 judges whether the time difference computed in step S705 is greater than the affected time (step S707). In a case in which it is judged that the time difference is greater than the affected time (YES in step S707), the server CPU 22 adds 1 to the counter J (step S708). In a case in which it is judged that the time difference is the affected time or less (NO in step S707), and after step S708 ends, the server CPU 22 judges whether the process ended (step S709). The server CPU 22 judges that the process ended in a case in which an instruction, indicating that the input of the event ended, is acquired via the network 19.

In a case in which it is judged that the process has not ended (NO in step S709), the server CPU 22 returns the process to step S703. In the case in which it is judged that the process ended (YES in step S709), the server CPU 22 creates a record recorded with an Ith acquired time, an Ith acquired event, the time difference computed in step S705, and the counter J, and records the record in the time-series DB 42 (step S710). The server CPU 22 thereafter ends the process.

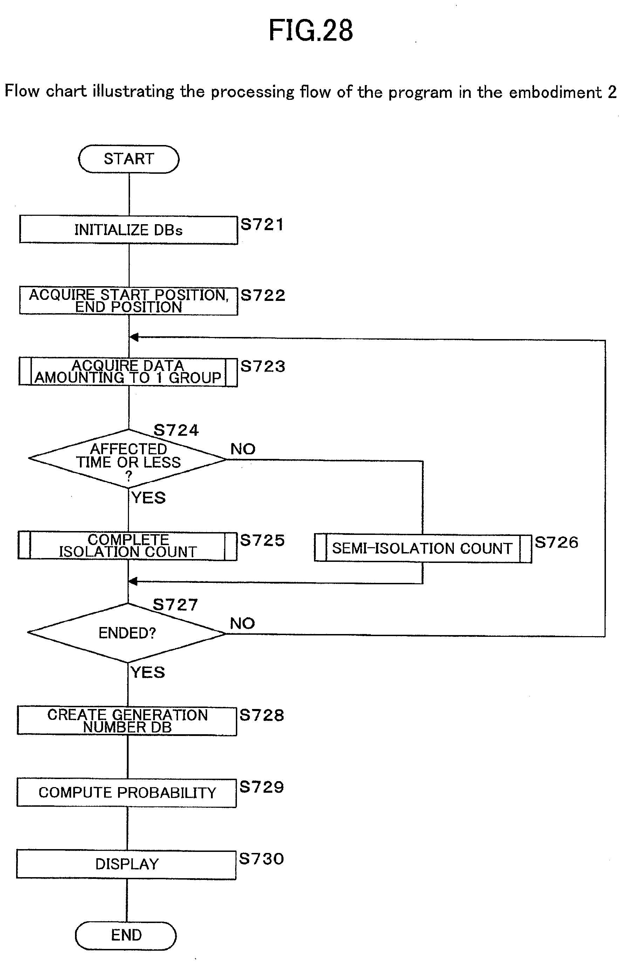

FIG. 28 is a flow chart illustrating a processing flow of the program in the embodiment 2. The program illustrated in FIG. 28 is a program that acquires the record from the time-series DB 42 illustrated in FIG. 26, and computes the generation probability of the combination of 2 events within the affected time. The processing flow in this embodiment will be described, by referring to FIG. 28.

The server CPU 22 initializes the combination count DB 43 and the combination time DB (step S721). In other words, the server CPU 22 sets the value of each field of the combination count DB 43 to an initial value, which is 0. In addition, the server CPU 22 deletes all of the records in the combination time DB.

The server CPU 22 acquires the start position and the end position of the analysis within the time-series DB 42 (step S722). The server CPU 22 acquires the start position and the end position of the analysis, from the input made from the input part 36 by the user such as the physician or the like who analyzes the state of the subject, via the communication part 25, the network 19, and the communication part 35. The server 22 acquires the data amounting to 1 group by referencing the group field of the time-series DB 42 (step S723). The data amounting to 1 group means the data in the records having the group fields recorded with the same value.

The server CPU 22 judges whether a time between the heat event and the last event in the acquired group is the affected time or shorter (step S724). In a case in which the time is the affected time or shorter (YES in step S724), the server CPU 22 boots a subroutine for complete isolation count (step S725). The subroutine for complete isolation count is a subroutine that records the combination of events within the complete isolation set in the combination count DB 43. In this embodiment, the subroutine for complete isolation count uses the same subroutine as the subroutine described in conjunction with FIG. 16.

In a case in which the time is longer than the affected time (NO in step S724), the server CPU 22 boots a subroutine for semi-isolation count (step S726). The subroutine for semi-isolation count is a subroutine that records the combination of events within the semi-isolation set in the combination count DB 43. In this embodiment, the subroutine for semi-isolation count uses the same subroutine as the subroutine described in conjunction with FIG. 17.

After step S725 or step S726 ends, the server CPU 22 judges whether the analysis of the time-series DB 42 ended (step S727). The server CPU 22 acquires the end position of the analysis in step S722, as described above. In a case in which it is judged that the analysis has not ended (NO in step S727), the server CPU 22 returns the process to step S723. In a case in which it is judged that the analysis ended (YES in step S727), the server CPU 22 creates the generation number DB 44 by counting the number of events between the start time and the end time acquired in step S722 (step S728). The server CPU 22 computes the probability that the second event is generated when the first event is generated, from the combination count DB 43 and the generation number DB 44, and records the computed probability in the combination probability DB 45 (step S729). The server CPU 22 displays the combination probability DB 45 or the like created by the processes described above, on the display part. 37 of the client 30, via the communication part 25, the network 19, and the communication part 35 (step S730). For example, the screens illustrated in FIGs. 20 through 22 are examples of the display that is made. Thereafter, the server CPU 22 ends the process.

According to this embodiment, when recording information in the time-series DB 42, the events are divided into groups of events separated by the affected time or longer, and thus, the server CPU 22 can perform a high-speed operation when the physician or the like thereafter analysis the data.

### [Embodiment 3]

This embodiment relates to an information processing apparatus or the like that processes the semi-isolation set by dividing the semi-isolation set into complete isolation sets, by ignoring a part of the events. A description of the parts in common with the embodiment 1 will be omitted.

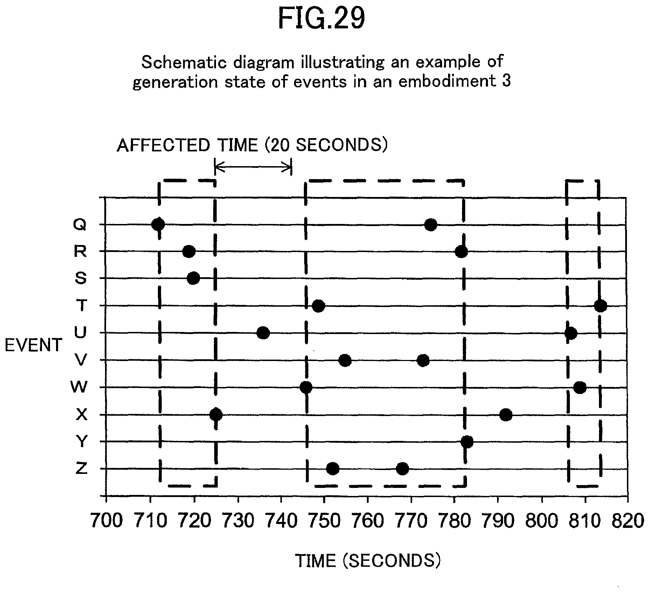

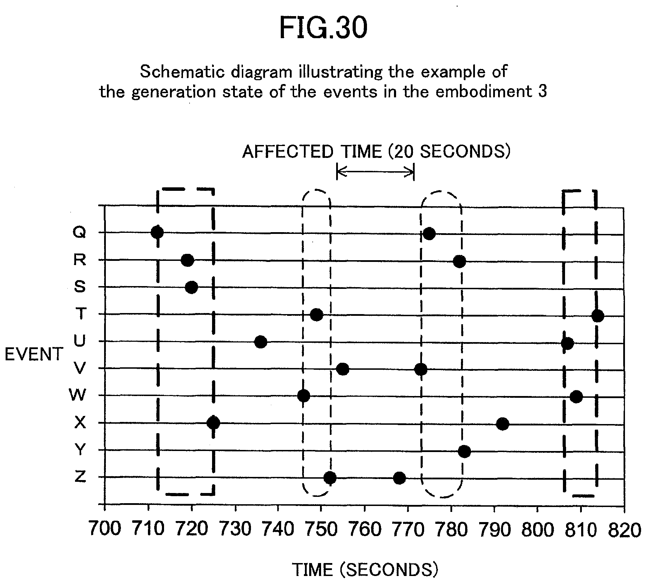

FIGs. 29 and 30 are schematic diagrams illustrating an example of the generation state of events in the embodiment 3. An abscissa of FIGs. 29 and 30 indicates the time. An ordinate of FIGs. 29 and 30 indicates the symbol of the generated event. A black circle indicates that the event indicated on the ordinate is generated at the time on the abscissa. A general description of this embodiment will be given, by referring to FIGs. 29 and 30.

FIG. 29 illustrates the generation state of events in a single semi-isolation set. Among the events illustrated in FIG. 29, the adjacent events are separated by the affected time of 20 seconds or less. In addition, the event at a left end of FIG. 29 and a previous event preceding the event at the left end are separated by longer than the affected time. Similarly, the event at a right end of FIG. 29 and a following event subsequent to the event at the right end are separated by longer than the affected time.

In a case in which 1 event is ignored, a state in which the semi-isolation set is divided so that an interval between an event preceding the ignored event and an event subsequent to the ignored event is longer than the affected time, is indicated by rectangular frames represented by a dotted line in FIG. 29. In a case in which a 5th event U and a 15th event X are ignored, the semi-isolation set can be divided into 3 groups separated by more than the affected time. Among the 3 groups, the group at the right end and the group at the left end are groups shorter than the affected time. On the other hand, the group at a center is a group longer the affected time. In the following description, the event that is ignored is referred to as a boundary event.

FIG. 30 illustrates a state in which the center group illustrated by the dotted line in FIG. 29 is further divided into two. FIG. 30 divides the events into groups that are separated by more than the affected time, in a case in which 2 consecutive events are boundary events. The further divided groups are indicated by elliptical frames represented by a dotted line in FIG. 30.

By the processes described above, the semi-isolation set illustrated in FIGs. 29 and 30 can be divided into 4 complete isolation sets and 4 boundary events. Suppose that a further divided group longer than the affected time is formed even in a case in which 2 consecutive events are regarded as the boundary events. In this case, the number of boundary events can be increased by regarding 3 events that are consecutive only at the part of this further divided group as the boundary events. Finally, all of the events included in the semi-isolation set can be divided into the complete isolation set and the boundary events. A DB used for this dividing operation will be described later.

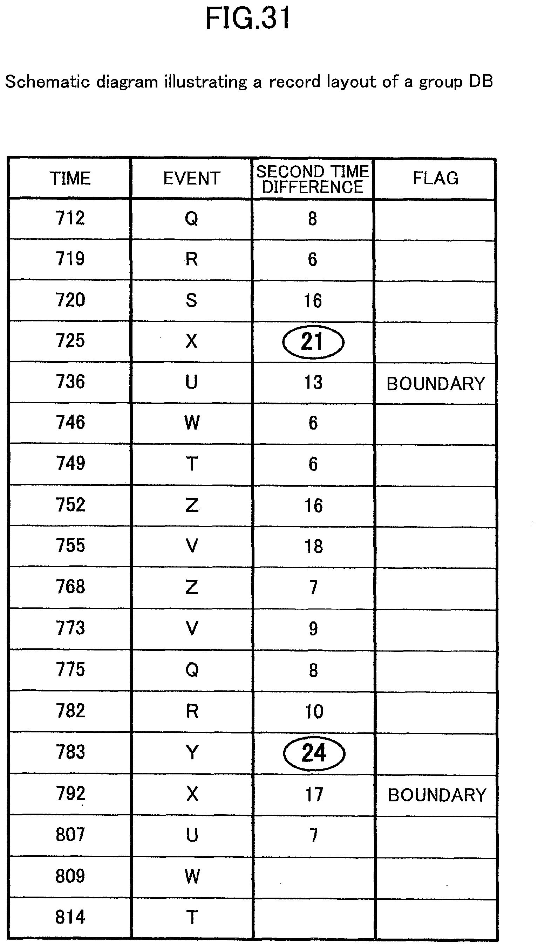

FIG. 31 is a schematic diagram illustrating a record layout of a group DB in the embodiment 3. The group DB is a DB that is used when dividing the semi-isolation set into the complete isolation sets and the boundary events. The group DB includes a time field, an event field, a second time difference field, and a flag field.

The time field is recorded with a numerical value indicating the date and time. The event field is recorded with a symbol indicating the change of state of the subject generated at the time recorded in the time field. The second time difference field is recorded with a time difference between an event and an event that is 2 events thereafter. The flag field is recorded with a flag indicating that the event is a boundary event. The group DB includes 1 record for 1 time corresponding to the event. The group DB is a DB that is temporarily created by the server CPU 22 if needed, in the auxiliary storage device 24 or the main storage device 23.

The server CPU 22 copies a part within the time-series DB 42, corresponding to the semi-isolation set on which the process of this embodiment is to be performed, to the time field and the event field. The group DB is also recorded in the ascending order of the data in the time fields, similarly to the time-series DB 42.

The server CPU 22 records, in the second time difference field, a difference between the value recorded in the time field and the value recorded in the time field of the record that is two records thereafter. For example, in the case of the record indicated at the top in FIG. 31, 712 is recorded in the time field. Two records thereafter, that is, in the record indicated third from the top, 720 is recorded in the time field. Accordingly, the server CPU 22 records, in the second time difference field of the record indicated at the top, 8, which is the difference between 720 and 712. The second time difference fields in the 2 last records in the group DB are blank, because a record that is two records thereafter does not exist in the group DB.

Among the second time difference fields of the group DB of FIG. 31, values exceeding the affected time of 20 seconds are recorded at 2 locations, as indicated by ovals. The server CPU 22 records "boundary", which indicates a boundary event, in the flag field of the record next to the record recorded with the value exceeding the affected time in the second time difference field.

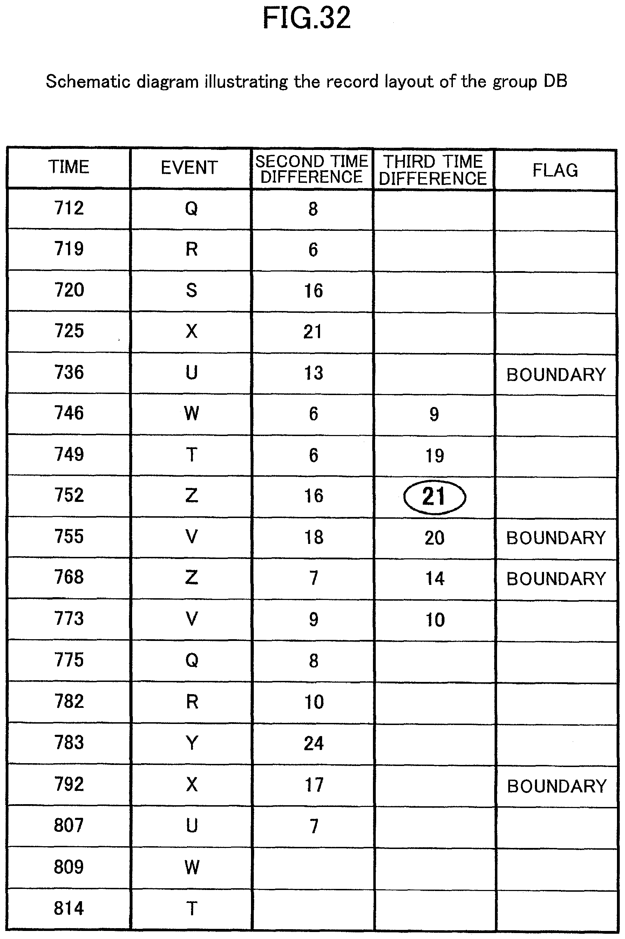

FIG. 32 is a schematic diagram illustrating a record layout of the group DB in the embodiment 3. The group DB illustrated in FIG. 32 is a DB in which a third time difference field is added to the group DB illustrated in FIG. 31.

The server CPU 22 regards, as the processing target, only the part in which a difference between the values recorded in the time field of the first record and the the time field of the last record in the group divided by the boundary events exceeds the affected time, that is, only the record included in the center group. The server CPU 22 records, in the third time difference field of the record that is the processing target, the difference between the values recorded in the time field of a record of interest and a record that is three records thereafter. For example, in the case of the record of interest having the time field recorded with 746 in FIG. 32, 755 is recorded in the time field of the record that is three records after the record of interest. Accordingly, the server CPU 22 records, in the third time difference field of the record of interest having the time recorded with 746, the difference between 755 and 746, which is 9. The third time difference fields in the 3 last records in the processing target are blank, because a record that is three records thereafter does not exist in the processing target.

Among the third time difference fields of the group DB of FIG. 32, a value exceeding the affected time of 20 seconds is recorded at 1 location, as indicated by an oval. The server CPU 22 records "boundary", which indicates a boundary event, in the flag field of 2 consecutive records next to the record recorded with the value exceeding the affected time in the third time difference field.

The group DB may be initially provided with time difference fields subsequent to the third time difference field. In this case, it is unnecessary to add a field in the DB during the process.

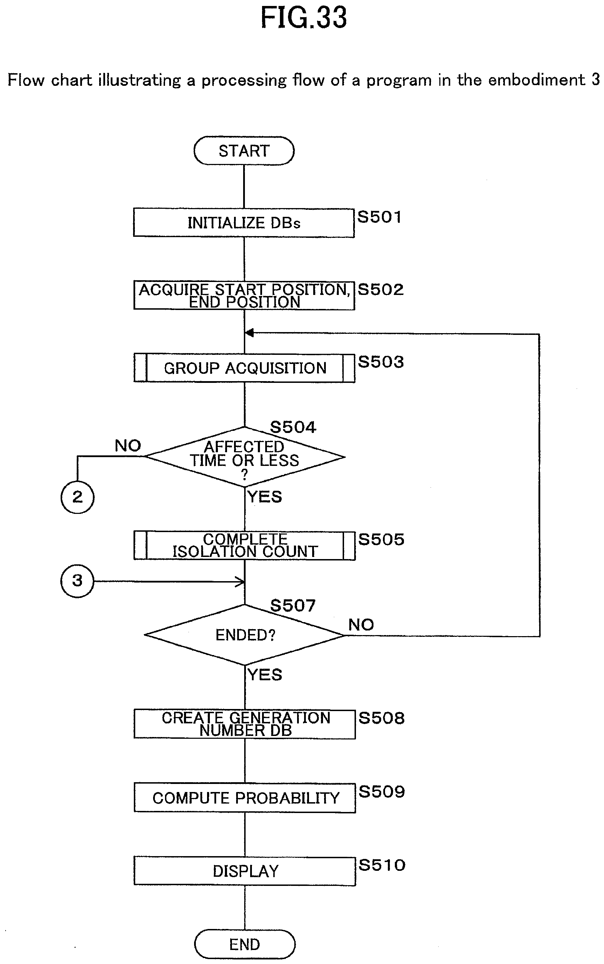

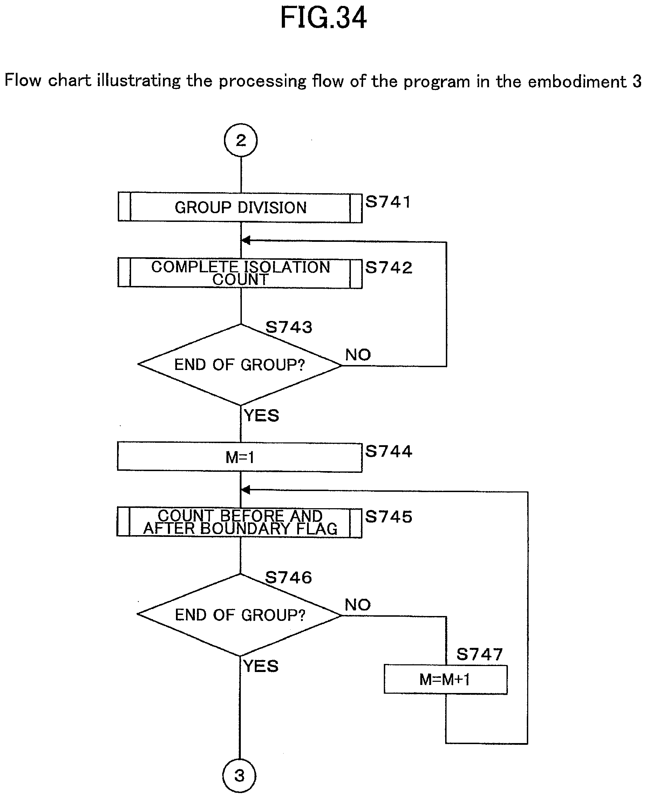

FIGs. 33 and 34 are flow charts illustrating a processing flow of a program in the embodiment 3. The processing flow of the program in this embodiment will be described, by referring to FIGs. 33 and 34. Step S501 through step S505, and step S507 through step S510 are the same as in the flow chart of the embodiment 1 described in conjunction with FIG. 14, and a description thereof will be omitted.

In a case in which the time between the head event and the last event of the acquired group exceeds the affected time (NO in step S504), the server CPU 22 boots a subroutine for group division (step S741). The subroutine for group division is a subroutine that divides the group acquired in step S503 by determining boundary events, as described in conjunction with FIGs. 29 through 31. The processing flow of the subroutine for group division will be described later.

The server CPU 22 boots a subroutine for complete isolation count (step S742). The subroutine for complete isolation count is a subroutine that records the combination of events within the complete isolation set in the combination count DB 43. In this embodiment, the subroutine for complete isolation count uses the same subroutine as the subroutine described in conjunction with FIG. 16.

The server CPU 22 judges whether the group acquired in step S503 is processed to the end of the group (step S743). In a case in which it is judged that the group is not processed to the end (NO in step S743), the server CPU 22 returns the process to step S742. In a case in which it is judged that the group is processed to the end (YES in step S743), the server CPU 22 sets the counter M to an initial value, which is 1 (step S744).

The server CPU 22 boots a subroutine for counting before and after the boundary flag (step S745). The subroutine for counting before and after the boundary flag is a subroutine that records, in the combination count DB 43, the combination of a Mth boundary event and events before and after the Mth boundary event and within the range of the affected time. The processing flow of the subroutine for counting before and after the boundary flag will be described later.

The server CPU 22 judges whether the group acquired in step S503 is processed to the end boundary event (step S746). In a case in which it is judged that the group is not processed to the end boundary event (NO in step S746), the server CPU 22 adds 1 to the counter M (step S747). The server CPU 22 returns the process to step S745. In a case in which it is judged tat the group is processed to the end boundary event (YES in step S746), the server CPU 22 advances the process to step S507.

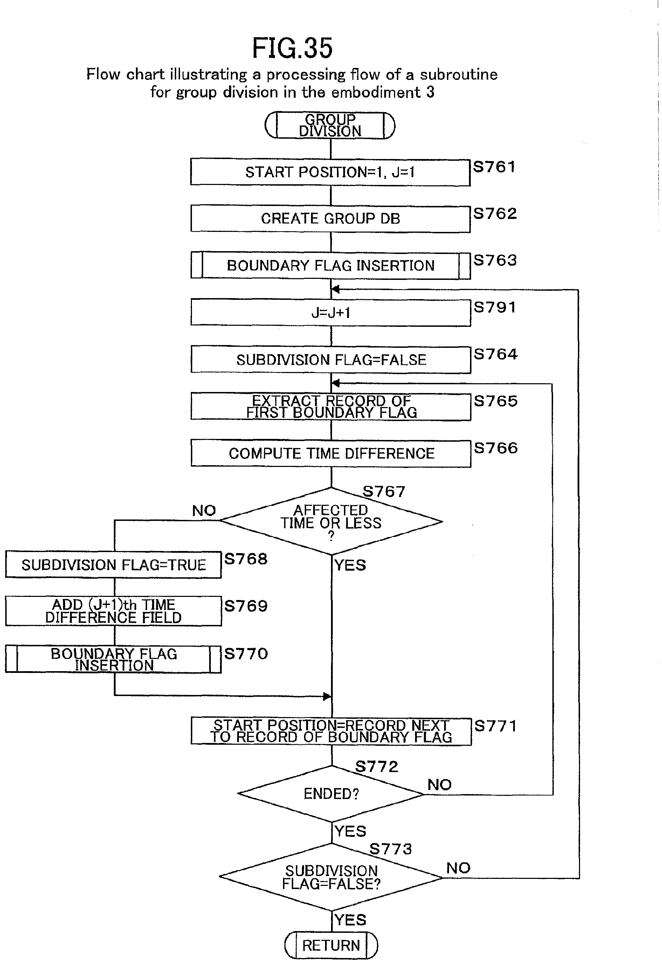

FIG. 35 is a flow chart illustrating a processing flow of a subroutine for group division in the embodiment 3. The subroutine for group division is a subroutine that divides the group by determining the boundary events. The processing flow of the subroutine for group division will be described, by referring to FIG. 35.

The server CPU 22 sets the start position to 1, and the counter J to 1 (step S761). The start position is a counter that indicates a reference record of the series of processes within the group DB. The counter J is a counter that indicates the number of boundary flags to be consecutively inserted. The server CPU 22 creates the group DB having the records corresponding to the group acquired in step S503 (step S762). The record layout of the group DB that is created is the record layout described in conjunction with FIG. 31. The server CPU 22 boots a subroutine for boundary flag insertion (step S763). The subroutine for boundary flag insertion is a subroutine that inserts a flag indicating the boundary event into the boundary field of the group DB. The processing flow of the subroutine for boundary flag insertion will be described later. The processing target of the subroutine for boundary flag insertion, booted in step S763, is all of the records included in the group acquired in step S503.

The server CPU 22 adds 1 to the counter J (step S791). The server CPU 22 sets a subdivision flag to an initial value, which is FALSE (step S764). The server CPU 22 searches the boundary fields of the group DB, and extracts the record recorded with the first boundary flag counted from the start position (step S765). The server CPU 22 computes a time difference, from a difference between the value recorded in the time field of the record that is 1 record before the record extracted in step S765, and the value recorded in the time field of the record corresponding to the start position (step S766).

The server CPU 22 judges whether the time difference is the affected time or less (step S767). In a case in which it is judged that the time difference exceeds the affected time (NO in step S767), the server CPU 22 sets a subdivision flag to TRUE (step S768). The server CPU 22 adds a J+1 time difference field to the group DB (step S769). For example, in a case in which J is 2, the server CPU 22 adds a third time difference field in step S769. The server CPU 22 records, in the added J+1 time difference field, a difference between the value recorded in the time field and the value recorded in the time field of the record that is J+1 records thereafter.

The server CPU 22 boots a subroutine for boundary flag insertion (step S770). The subroutine for boundary flag insertion is a subroutine that is identical to the subroutine booted in step S763. The processing target of the subroutine for boundary flag insertion, booted in step S770, is the record corresponding to a start flag up to the record that is 1 record before the record extracted in step S765.

After step S770 ends or in a case in which it is judged that the time difference is the affected time or less (YES in step S767), the server CPU 22 sets the start position to the number that indicates the record next to the record having the first boundary flag extracted in step S765 (step S771).

The server CPU 22 judges whether the processing of the group ended (step S772). In a case in which it is judged that the processing of the group has not ended (NO in step S772), the server CPU 22 returns the process to step S765. In a case in which it is judged that the process of the group ended (YES in step S772), the server CPU 22 judges whether the subdivision flag is FALSE (step S773). In a case in which the subdivision flag is not FALSE, that is, the subdivision flag is TRUE (NO in step S773), the server CPU 22 returns the process to step S791. In a case in which it is judged that the subdivision flag is FALSE (YES in step S773), the server CPU 22 ends the process.

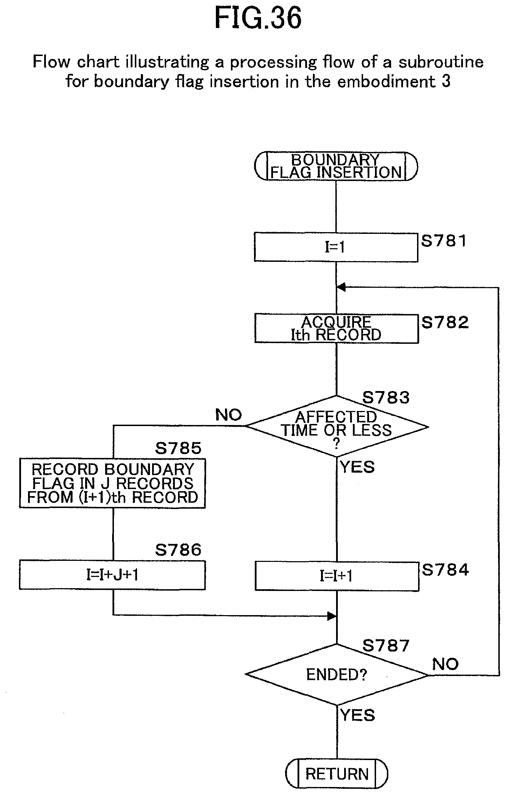

FIG. 36 is a flow chart illustrating a processing flow of a subroutine for boundary flag insertion in the embodiment 3. The subroutine for boundary flag insertion is a subroutine that inserts a flag indicating the boundary event into the boundary field of the group DB. The processing flow of the subroutine for boundary flag insertion will be described, by referring to FIG. 36.

The server CPU 22 sets the counter I to an initial value, which is 1 (step S781). The server CPU 22 acquires an Ith record from the records that are processing target of this subroutine within the group DB (step S782). The server CPU 22 judges whether a largest value in the time difference field of the acquired record is the affected time or less (step S783). In other words, in a case in which time difference fields up to the second time difference field exist, a judgement is made to determine whether the value in the second time difference field is the affected time or less. In a case in which time difference fields up to the third time difference fields exist, a judgment is made to determine whether the value in the third time difference field is the affected time or less.

In a case in which it is judged that the value in the time difference field is the affected time or less (YES in step S783), the server CPU 22 adds 1 to the counter I (step S784). In a case in which it is judged that the value in the time difference field exceeds the affected time (NO in step S783), the server CPU 22 records the boundary flag in the flag fields of J records from the (I+1)th record (step S785). In this case, J is a counter that is acquired as an argument from a calling source program of this subroutine, and indicates the number of boundary flags to be consecutively inserted. The server CPU 22 adds J+1 to the counter I (step S786).

After step S784 or step S786, the server CPU 22 judges whether processing of the record ended (step S787). In a case in which it is judged that the processing of the record has not ended (NO in step S787), the server CPU 22 returns the process to step S782. In a case in which it is judged that the processing of the record ended (YES in step S787), the server CPU 22 ends the process.

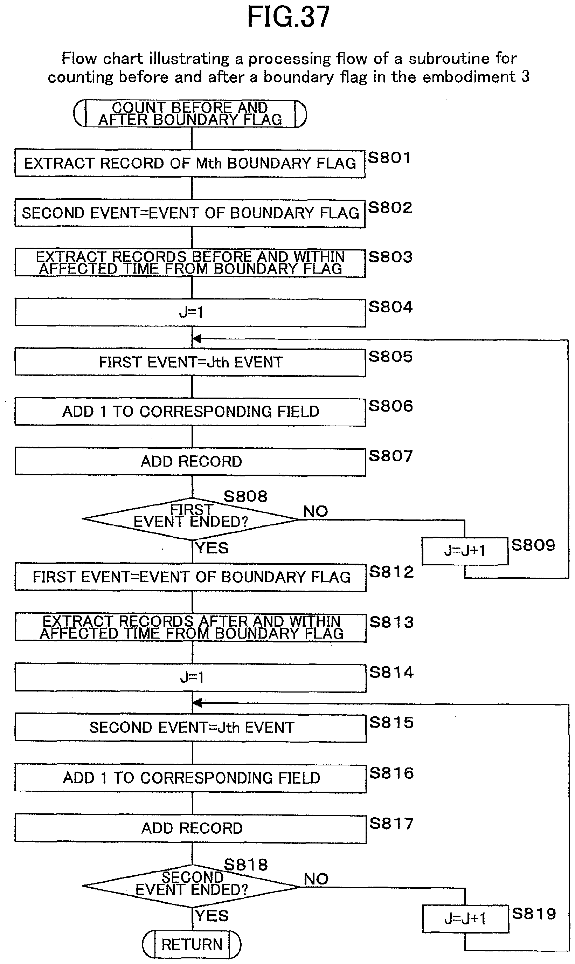

FIG. 37 is a flow chart illustrating a processing flow of a subroutine for counting before and after a boundary flag in the embodiment 3. The subroutine for counting before and after the boundary event is a subroutine that records, in the combination count DB 43, the combination of the events within the range of the affected time before and after a Mth boundary event. The processing flow of the subroutine for counting before and after the boundary flag will be described, by referring to FIG. 37.

The server CPU 22 extracts the record including the Mth boundary flag (step S801). In this case, M is a counter that is acquired as an argument from a calling source program of this subroutine, and indicates the boundary flag of the processing target.

The server CPU 22 sets, in the second event, the event recorded in the event field of the extracted field (step S802). The second event is a name of a variable used within this subroutine. The server CPU 22 extracts records that are before and are within the range of the affected time from the record including the boundary flag, that is, extracts records of the past (step S803). The server CPU 22 sets the counter J to an initial value, which is 1 (step S804).

The server CPU 22 sets, in the first event, the event recorded in the event field of the Jth record among the records extracted in step S803 (step S805). The first event is a name of a variable used within this subroutine.

The server CPU 22 adds 1 to the field corresponding to the first event and the second event of the combination count DB 43 (step S806). More particularly, in a case in which the first event is B and the second event is C, 1 is added to the B-field of the record having C recorded in the second event. The server CPU 22 adds to the combination time DB a record recorded with the first event, the second event, and the time related to the second event (step S807).

The server CPU 22 judges whether the processing of the record of the first event extracted in step S803 ended (step S808). In a case in which it is judged that the processing of the record has not ended (NO in step S808), the server CPU 22 adds 1 to the counter J (step S809). Thereafter, the server CPU 22 returns the process to step S805.

In a case in which it is judged that the processing of the record ended (YES in step S808), the server CPU 22 sets, to the firs event, the event recorded in the event field of the record extracted in step S801 (step S812). The server CPU 22 extracts records that are within the range of the affected time from the record including the boundary flag, that is, extracts records of the future (step S813). The server CPU 22 sets the counter J to an initial value, which is 1 (step S814).

The server CPU 22 sets, to the second event, the event recorded in the event field of the Jth record among the records extracted in step S813 (step S815). The first event is a name of a variable used within this subroutine.

The server CPU 22 adds 1 to the field corresponding to the first event and the second event of the combination count DB 43 (step S816). The server CPU 22 adds to the combination time DB a record recorded with the first event, the second event, and the time related to the second event (step S817).

The server CPU 22 judges whether the processing of the record of the second event extracted in step S813 ended (step S818). In a case in which it is judged that the processing of the record has not ended (NO in step S818), the server CPU 22 adds 1 to the counter J (step S819). In a case in which it is judged that the processing of the record ended (YES in step S818), the server CPU 22 ends the process.

According to this embodiment, it is possible to provide the information processing system 10 having the short computation time, in a case in which an average value of the time interval between the events within the semi-isolation set falls slightly below the affected time.

An upper limit value of the counter J within the subroutine for the group division described in conjunction with FIG. 35 may be determined in advance. In this case, in a case in which J reaches the upper limit value, the group division process stops even when a group longer than the affected time remains. The combination within the group longer than the affected time is processed by the subroutine for semi-isolation count described in conjunction with FIG. 17. By appropriately setting J, it is possible to provide the information processing system 10 having the short computation time.

### {Embodiment 4]

This embodiment relates to an information processing apparatus or the like that determines a minimum number of consecutive boundary events, by adding the interval between the events from high ranked intervals. A description of the parts in common with the embodiment 3 will be omitted.

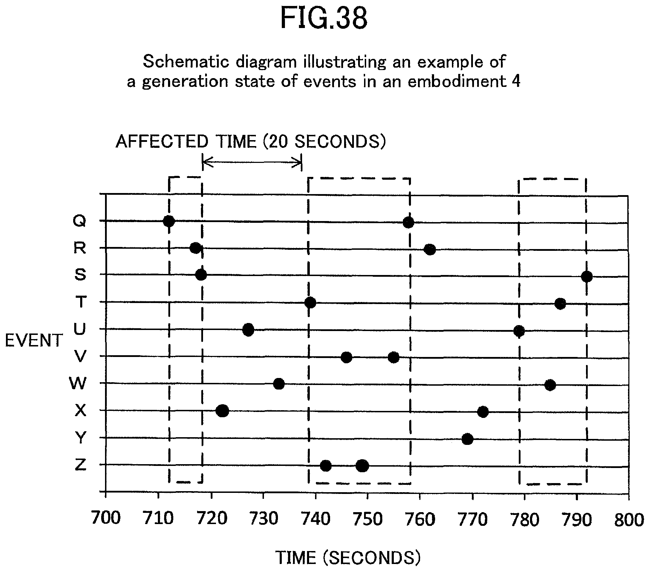

FIG. 38 is a schematic diagram illustrating an example of the generation state of events in the embodiment 4. An abscissa of FIG. 38 indicates the time. An ordinate of FIG. 38 indicates a symbol of the generated event. A black circle indicates that the event indicated on the ordinate is generated at the time on the abscissa. A general description of this embodiment will be given, by referring to FIG. 38.

FIG. 38 illustrates the generation state of events in a single semi-isolation set. Among the events illustrated in FIG. 38, the adjacent events are separated by the affected time of 20 seconds or less. In addition, the event at a left end of FIG. 38 and a previous event preceding the event at the left end are separated by longer than the affected time. Similarly, the event at a right end of FIG. 38 and a following event subsequent to the event at the right end are separated by longer than the affected time.

In the case of FIG. 38, an unevenness of the interval between the events is small, and thus, the events cannot be divided into groups separated by more than the affected time, even when 1 event or 2 consecutive events are made boundary events. As indicated by a dotted line in FIG. 38, 3 consecutive events are made boundary events, so that the events can be divided into 3 groups separated by an interval longer than the affected time. In this case, it is possible to reduce the computation time, by adding the interval between the events from the high ranked intervals to determine the minimum number of required consecutive boundary events. A description will be given of the DBs used by the operation described above.

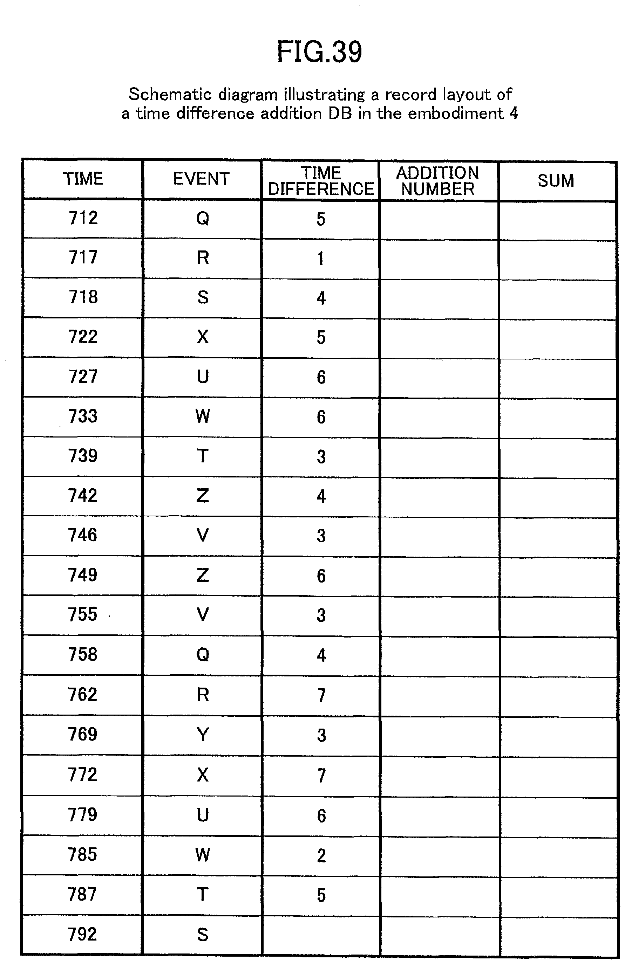

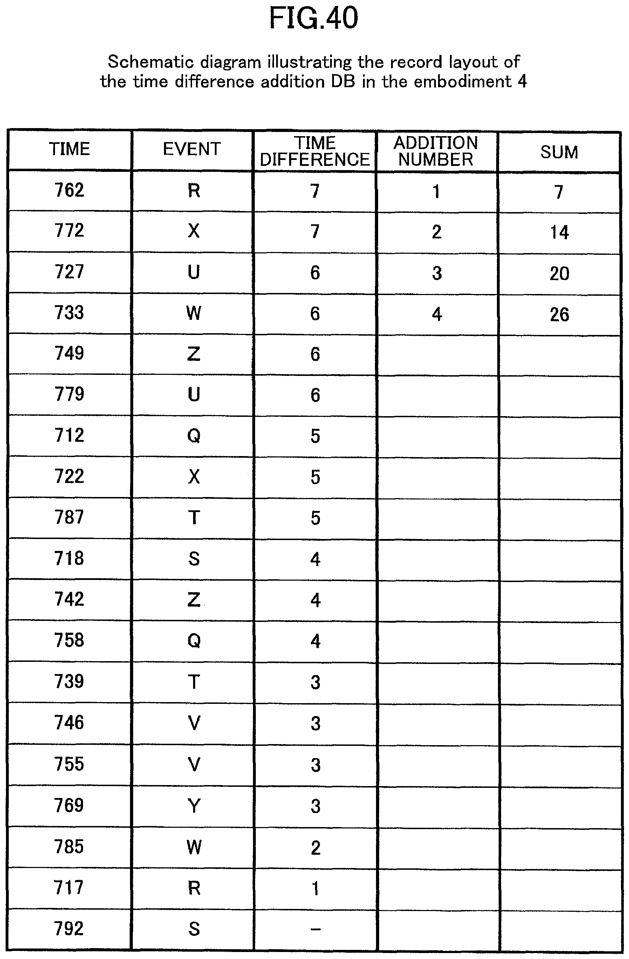

FIGs. 39 and 40 are schematic diagram illustrating a record layout of a time difference addition DB in the embodiment 4. The time difference addition DB is a DB that is used when dividing the semi-isolation set into the complete isolation sets and the boundary events. The time difference addition DB in this embodiment includes a time field, an event field, a time difference field, an addition number field, and a sum field. The time difference addition DB is a DB that is used when determining the number of consecutive boundary flag insertions after which the subroutine for boundary flag insertion is to be started.

The time field is recorded with a numerical value indicating the date and time. The event field is recorded with a symbol indicating a change in the state of the subject generated at the time recorded in the time field. The time difference field is recorded with a time difference between an event and a next event. The addition number field and the sum number field will be described later. The time difference addition DB includes 1 record for 1 time corresponding to the event. The time difference addition DB is a DB that is temporarily created by the server CPU 22 if needed, in the auxiliary storage device 24 or the main storage device 23.

The server CPU 22 copies a part within the time-series DB 42, corresponding to the semi-isolation set on which the process of this embodiment is to be performed, to the time field and the event field. The time difference addition DB illustrated in FIG. 39 is recorded in the ascending order of the data in the time fields.

The server CPU 22 records, in the time difference field, a difference between the value recorded in the time field and the value recorded in the time field of the next record. For example, in the case of the record indicated at the top in FIG. 39, 712 is recorded in the time field. In the next record, 717 is recorded in the time field. Accordingly, the server CPU 22 records, in the time difference field of the record indicated at the top, 5, which is the difference between 717 and 712. The time difference field in the last 1 record in the group DB is blank, because a next record does not exist in the group DB.

The server CPU 22 rearranges the records in the descending order using, as a key, the value recorded in the time difference field. An example of the group DB after the rearrangement is illustrated in FIG. 40. The server CPU 22 records 0 in the addition number field of the top record, that is, the record having the largest value recorded in the time difference field. The server CPU 22 records, in the sum field of the same record, a value obtained by adding the values recorded in a number of higher ranked time difference fields indicated by the value recorded in the addition number field.

The server CPU 22 successively adds 1 to the numerical value recorded in the addition number field, and successively obtains the value to be recorded in the sum field. For example, in a case of the record in which the value of the time field is 733, the value of the addition number field is 4, and the value of the sum field is 26 which is obtained by adding the values of the time difference fields of 4 high ranked records. The value recorded in the sum field exceeds the affected time from this record in which the time field is 733, and thus, the server CPU 22 stops the processing of the addition number field and the sum field.

It may be understood from the description above that, in the case of this embodiment, 4 or more time differences between the events need to be added in order to exceed the affected time. This means that the number of boundary events is 3 or more. Accordingly, it is possible to reduce the computation time by starting the subroutine for boundary flag insertion described in conjunction with FIG. 36 from the insertion of 3 consecutive boundary flags.

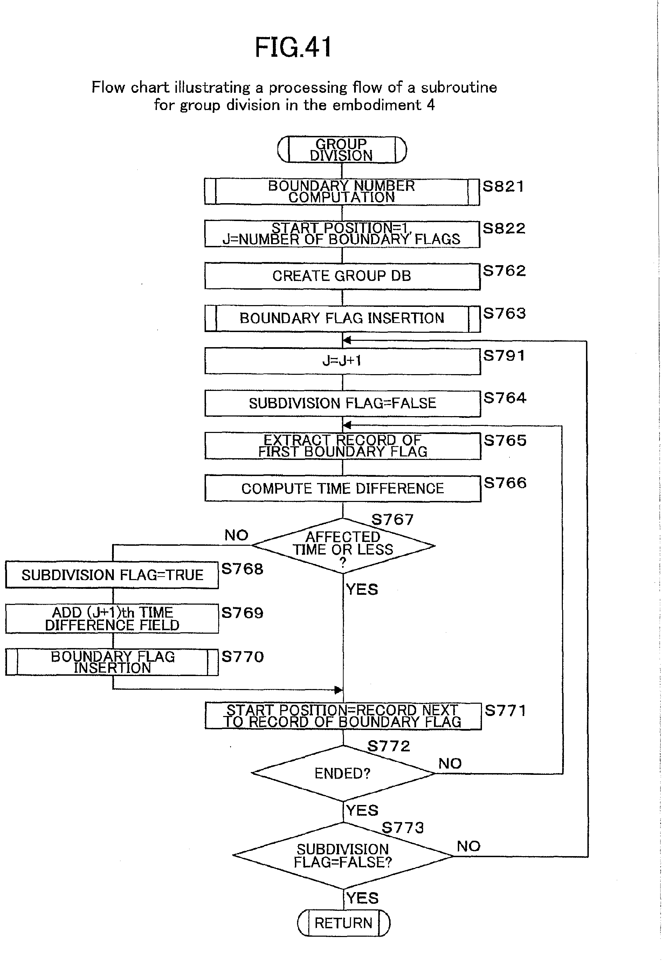

FIG. 41 is a flow chart illustrating a processing flow of a subroutine for group division in the embodiment 4. This subroutine is a subroutine that is used in place of the subroutine for group division described in conjunction with FIG. 35. The processing flow of the subroutine for group division in this embodiment will be described, by referring to FIG. 41.

The server CPU 22 boots a subroutine for boundary number computation (step S821). The subroutine for boundary number computation is a subroutine that determines the number of consecutive boundary flag insertions from which the subroutine for boundary flag insertion is to be started. A processing flow of the boundary number computation will be described later.

The server CPU 22 sets the start position to 1, and sets the counter J to a boundary flag number computed by the subroutine for boundary number computation (step S822). The start position is a counter that indicates a reference record of the series of processes within the group DB. The counter J is a counter that indicates the number of boundary flags to be consecutively inserted. The server CPU 22 creates the group DB (step S762). Processes of step S762 and subsequent steps are the same as the processes of the subroutine for group division in the embodiment 3 described in conjunction with FIG. 35, and a description thereof will be omitted.

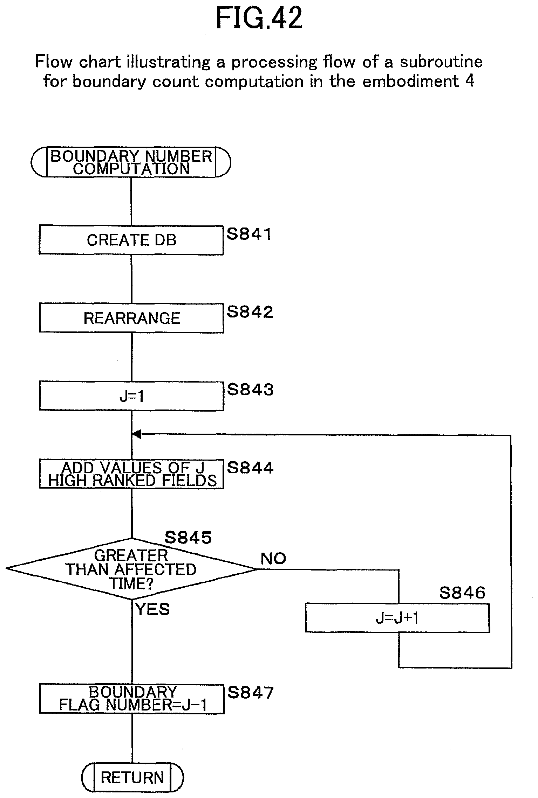

FIG. 42 is a flow chart illustrating a processing flow of the subroutine for boundary number computation in the embodiment 4. The subroutine for boundary number computation is a subroutine that determines the number of consecutive boundary flag insertions from which the subroutine for boundary flag insertion is to be started. The processing flow of the subroutine for boundary number computation will be described, by referring to FIG. 42.

The server CPU 22 creates a time difference addition DB having records corresponding to the groups acquired in step S593 (step S841). The time difference addition DB is the DB described in conjunction with FIGs. 39 and 40. The server CPU 22 copies a part corresponding to the semi-isolation set on which the process of this embodiment is to be performed, to the time field and the event field. In addition, the server CPU 22 records, in the time difference field of the time difference addition DB, the difference between the value recorded in the time field and the value recorded in the time field of the next record.

The server CPU 22 rearranges the records of the time difference addition DB in an descending order, using the time difference field as a key (step S842). The server CPU 22 sets the counter J to an initial value, which is 1 (step S843).

The server CPU 22 computes a total value by adding the values of J high ranked time difference fields (step S844). The server CPU 22 judges whether the computed total value is greater than the affected time (step S845). In a case in which it is judged that the computed total value is the affected time or less (NO in step S845), the server CPU 22 adds 1 to the counter J (step S846). The server CPU 22 returns the process to step S844. In a case in which it is judged that the computed total value is greater than the affected time (YES in step S845), the server CPU 22 sets the boundary flag number to J-1 (step S847). The boundary flag number is an argument used by this subroutine for argument passing to the calling source. The server CPU 22 thereafter ends the process.

### [Embodiment 5]

This embodiment relates to an information processing apparatus or the like that processes the semi-isolation set and the complete isolation set separately, by selecting, as the boundary event, the event having the time differences with the events that are before and after this event that become larger, and in a case in which the time differences of the events before and after this boundary event is less than the affected time, also selecting the adjacent event as the boundary event. A description of the parts in common with the embodiment 3 will be omitted.

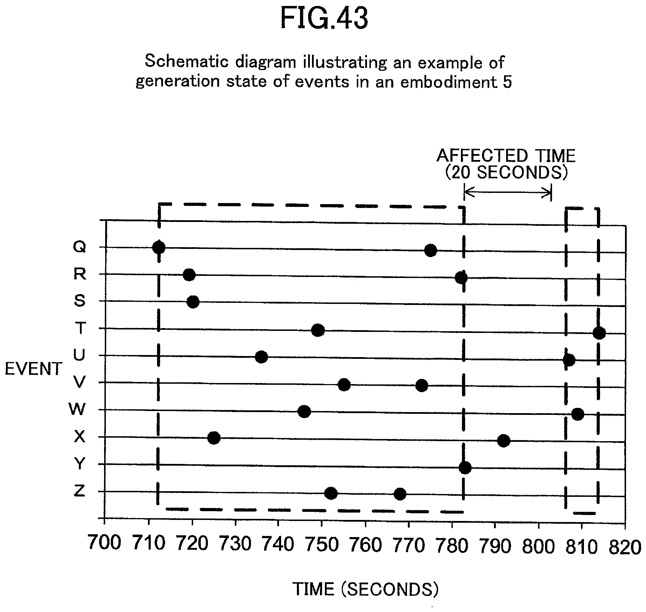

FIGs. 43 through 45 are schematic diagrams illustrating an example of the generation state of events in the embodiment 5. An abscissa of FIGs. 43 through 45 indicates the time. An ordinate of FIGs. 43 through 45 indicates the symbol of the generated event. A black circle indicates that the event indicated on the ordinate is generated at the time on the abscissa. A general description of this embodiment will be given, by referring to FIGs. 43 through 45.

FIGs. 43 through 45 illustrate the generation state of the events of a single semi-isolation set. Among the events illustrated in FIGs. 43 through 45, the adjacent events are separated by the affected time of 20 seconds or less. In addition, the event at a left end of FIGs. 43 through 45 and a previous event preceding the event at the left end are separated by longer than the affected time. Similarly, the event at a right end of FIGs. 43 through 45 and a following event subsequent to the event at the right end are separated by longer than the affected time.

In FIG. 43, the event having the longest interval from the event on the right side is selected as the boundary event. The semi-isolation set is separated by this boundary event into 2 groups indicated by rectangular frames represented by a dotted line. The interval between the 2 groups is longer than the affected time. The group on the right side is shorter than the affected time, and the group on the left side is longer than the affected time.

In FIG. 43, with respect to the group on the right side in FIG. 43, the event having the longest interval from the event on the right side and the event adjacent to the event having the longest interval are selected as the boundary event. The group on the right side is separated by this boundary event into 2 groups indicated by rounded rectangular frames represented by a dotted line. The interval between the 2 groups is longer than the affected time. The group on the right side is shorter than the affected time, and the group on the left side is longer than the affected time.

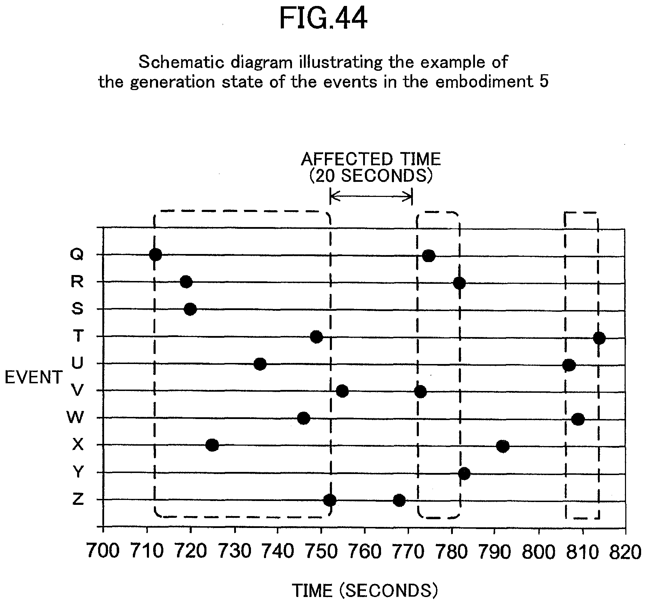

In FIG. 44, with respect to the group on the right side in FIG. 43, the event having the longest interval from the event on the right side the event adjacent to the event having the longest interval are selected as the boundary event. The group on the right side is separated by this boundary event into 2 groups indicated by rounded rectangular frames represented by a dotted line. The interval between the 2 divided groups is longer than the affected time. Between the 2 divided groups, the group on the right side is shorter than the affected time, and the group on the left side is longer than the affected time.

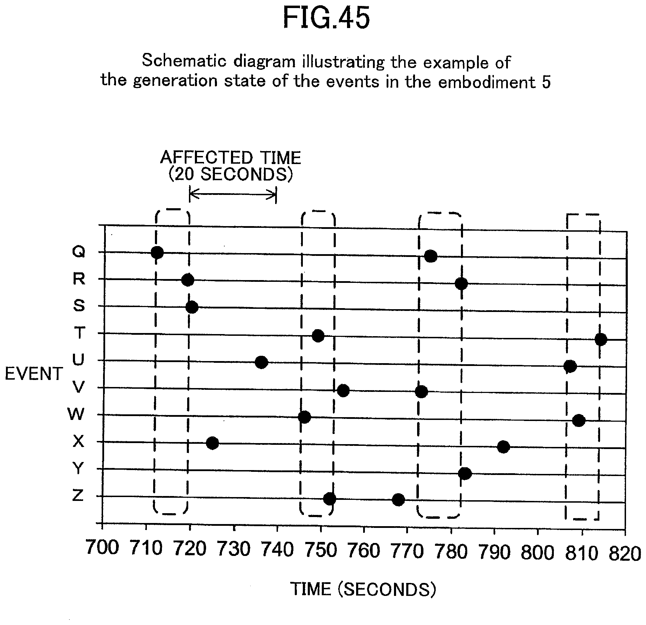

In FIG. 45, with respect to the group on the right side in FIG. 44, the event having the longest interval from the event on the right side and the event adjacent to the event having the longest interval are selected as the boundary event. The group on the right side is separated by this boundary event into 2 groups indicated by rectangular frames represented by a dotted line. The interval between the 2 divided groups is longer than the affected time. The 2 divided groups on the left and right sides are shorter than the affected time. Hence, the semi-isolation set illustrated in FIGs. 43 through 45 can be divided into 4 complete isolation sets and 5 boundary events.

Even when 2 consecutive events are boundary events, the number of consecutive boundary events is successively increased in a case in which the interval between the groups is shorter than the affected time. A description will be given of the DBs used by the division operation described above.

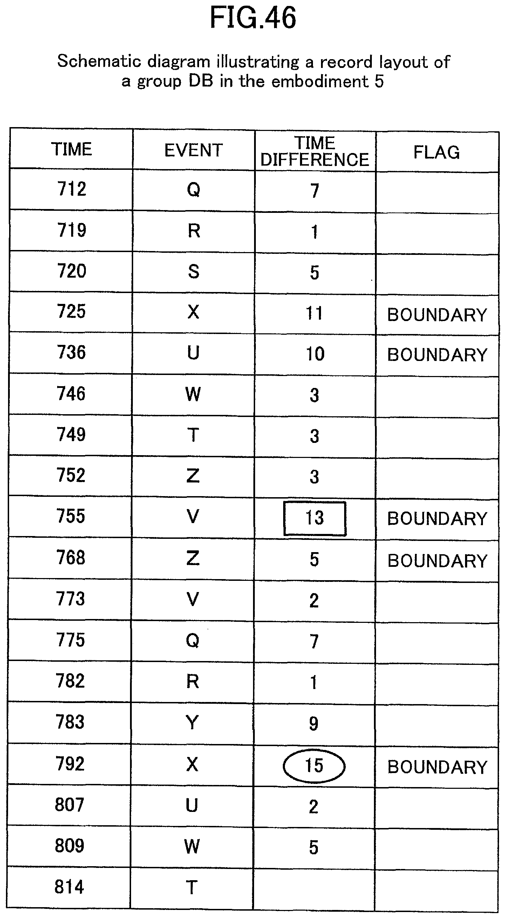

FIG. 46 is a schematic diagram illustrating a record layout of the group DB in the embodiment 5. The group DB is a DB that is used when dividing the semi-isolation set into the complete isolation sets and the boundary events. The group DB includes a time field, an event field, a time difference field, and a flag field.

The time field is recorded with a numerical value indicating the date and time. The event field is recorded with a symbol indicating a change in the state of the subject generated at the time recorded in the time field. The time difference field is recorded with a time difference between an event and an event that is 1 event thereafter. The flag field is recorded with a flag indicating that the event is a boundary event. The group DB includes 1 record for 1 time corresponding to the event. The group DB is a DB that is temporarily created created by the server CPU 22 if needed, in the auxiliary storage device 24 or the main storage device 23.

The server CPU 22 copies a part within the time-series DB 42, corresponding to the semi-isolation set on which the process of this embodiment is to be performed, to the time field and the event field. The group DB is also recorded in the ascending order of the data in the time fields, similarly to the time-series DB 42.

The server CPU 22 records, in the time difference field, a difference between the value recorded in the time field and the value recorded in the time field of the next record. For example, in the case of the record indicated at the top in FIG. 46, 712 is recorded in the time field. In the next record, that is, in the record indicated second from the top, 719 is recorded in the time field. Accordingly, the server CPU 22 records, in the time difference field of the record indicated at the top, 7, which is the difference between 719 and 712. The time difference field in the last records in the group DB is blank, because a next record does not exist in the group DB.

The server CPU 22 searches the time difference fields of the group DB of FIG. 46, and extracts a record with the largest value recorded in the time field. In the case of FIG. 46, the record having a value 15 recorded in the time difference field, as illustrated by an oval, is the record having the largest value recorded in the time field. Accordingly, the server CPU 22 records "boundary", which indicates a boundary event, in the flag field of this record having the largest value recorded in the time difference field. The time field of the record preceding the record of the boundary event is recorded with a value 783, and the time field of the record subsequent to the record of the boundary event is recorded with a value 807. Accordingly, the server CPU 22 judges that the interval between the groups before and after the boundary event is the difference of 807 and 783, which is 24, and that the interval of the 2 groups is longer than the affected time. In addition, because a length of the group on the subsequent side of the boundary event is the difference between 814 and 807 recorded in the time fields, which is 7, the server CPU 22 judges that the group on the subsequent side of the boundary event is shorter than the affected time. Similarly, because a length of the group on the preceding side of the boundary event is the difference between 783 and 712 recorded in the time fields, which is 71, the server CPU 22 judges that the group on the preceding side of the boundary event is longer than the affected time.

The server CPU 22 starts processing of the long group on the preceding side of the boundary event. The server CPU 22 searches the time difference fields within the long group on the preceding side of the boundary event, and extracts the record having the largest value. In the case of FIG. 46, the record having the largest value recorded in the time difference field is the record having a value 13 indicated by a rectangle recorded in the time difference field. Accordingly, the server CPU 22 records "boundary", which indicates the boundary event, in the flag field of this record. The time field of the record on the preceding side of the boundary event is recorded with a value 752, and the time field of the record on the subsequent side of the boundary event is recorded with a value 768. Hence, the server CPU 22 judges that the interval of the groups before and after the boundary field is 16, which is the difference between 768 and 752, and that the interval between the 2 groups is shorter than the affected time.

The server CPU 22 compares the values recorded in the time difference fields of the records before and after the record having a value 13 recorded in the time difference field, and records "boundary", which indicates the boundary event, in the flag field of the record having the larger value recorded in the time difference field. The time field of the record before the 2 boundary events is recorded with a value 752, and the time field of the record after the 3 boundary events is recorded with a value 773. Accordingly, the server CPU 22 judges that the interval of the groups before and after the 2 boundary events is 21, which is the difference between 773 and 752, and that the interval between the 2 groups is longer than the affected time. In addition, the server CPU 22 judges that the length of the group after the 2 boundary events is 6, which is the difference between 783 and 773, and that this group is shorter than the affected time. The server CPU 22 judges that the length of the group before the 2 boundary events is 40, which is the difference between 752 and 712, and that this group is longer than the affected time.

Thereafter, the server CPU 22 extracts the record having the largest value recorded in the time difference field within the group longer than the affected time and judges that the event of this record is the boundary event. The server CPU 22 repeats the operation of judging that the adjacent event is the boundary event until the interval between the groups becomes longer than the affected time. By the above described processes, the server CPU 22 divides the semi-isolation set into 4 complete isolation sets and 5 boundary events.

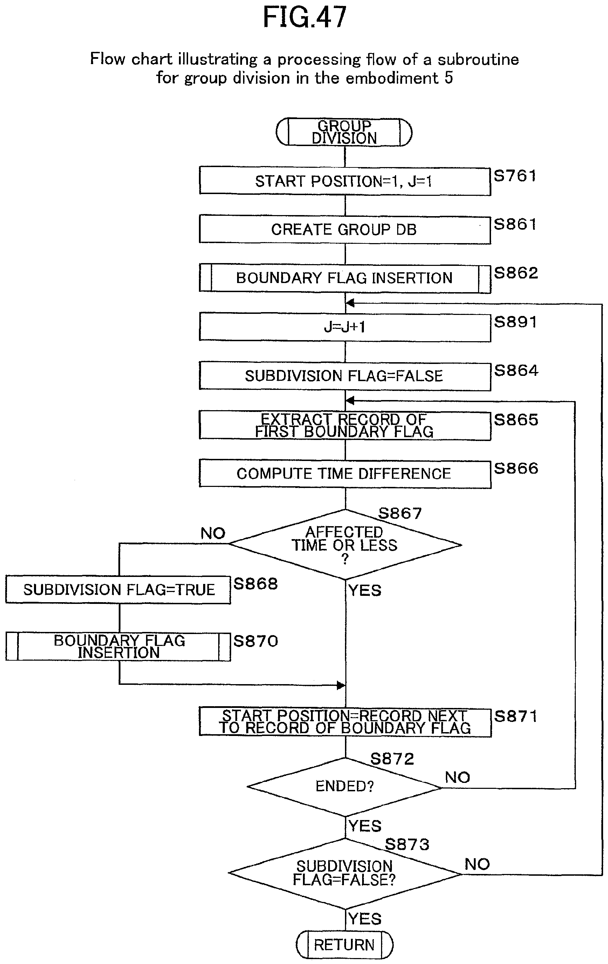

FIG. 47 is a flow chart illustrating a processing flow of a subroutine for group division in the embodiment 5. This subroutine is the subroutine that is used in place of the subroutine for group division described in conjunction with FIG. 35. The processing flow of the subroutine for group division in this embodiment will be described, by referring to FIG. 47.

The server CPU 22 sets the start position to 1, and the counter J to 1 (step S761). The start position is a counter that indicates the reference record of the series of processes within the group DB. The counter J is a counter that indicates the number of boundary flags to be consecutively inserted. The server CPU 22 creates the group DB having the records corresponding to the groups acquired in step S503 (step S861). The group DB that is created has the layout described in conjunction with FIG. 46. The server CPU 22 boots a subroutine for boundary flag insertion (step S862). The subroutine for boundary flag insertion is a subroutine that inserts a flag indicating the boundary event into the boundary field of the group DB. The processing flow of the subroutine for boundary flag insertion will be described later. The processing target of the subroutine for boundary flag insertion, booted in step S862, is all of the records included in the group acquired in step S503.

The server CPU 22 adds 1 to the counter J (step S891). The server CPU 22 sets a subdivision flag to an initial value, which is FALSE (step S864). The server CPU 22 searches the boundary fields of the group DB, and extracts the recorded recorded with the first boundary flag counted from the start position (step S865). The server CPU 22 computes a time difference from the difference between the value recorded in the time field of the record that is 1 record before the record extracted in step S765, and the value recorded in the time field of the record corresponding to the start position (step S866).

The server CPU 22 judges whether the computed time difference is the affected time or less (step S867). In a case in which it is judged that the computed time difference exceeds the affected time (NO in step S867), the server CPU 22 sets the subdivision flag to TRUE (step S868). The server CPU 22 boots a subroutine for boundary flag insertion (step S870). The subroutine for boundary flag insertion is a subroutine that is identical to the subroutine booted in step S862. The processing target of the subroutine for boundary flag insertion, booted in step S870, is the record corresponding to a start flag up to the record that is 1 record before the record extracted in step S865.

After step S870 or when the computed time difference is the affected time or less (YES in step S867), the server CPU 22 sets the start position to the number indicating the record that is next to the record having the first boundary flag extracted in step S865 (step S871).

The server CPU 22 judges whether the processing of the group ended (step S872). In a case in which it is judged that the processing of the group has not ended (NO in step S872), the server CPU 22 returns the process to step S865. In a case in which it is judged that the processing of the group ended (YES in step S872), the server CPU 22 judges whether a subdivision flag is FALSE (step S873). In a case in which it is judged that the subdivision flag is not FALSE, that is, the subdivision flag is TRUE (No in step S873), the server CPU 22 returns the process to step S891. In a case in which it is judged that the subdivision flag is FALSE (YES in step S873), the server CPU 22 ends the process.

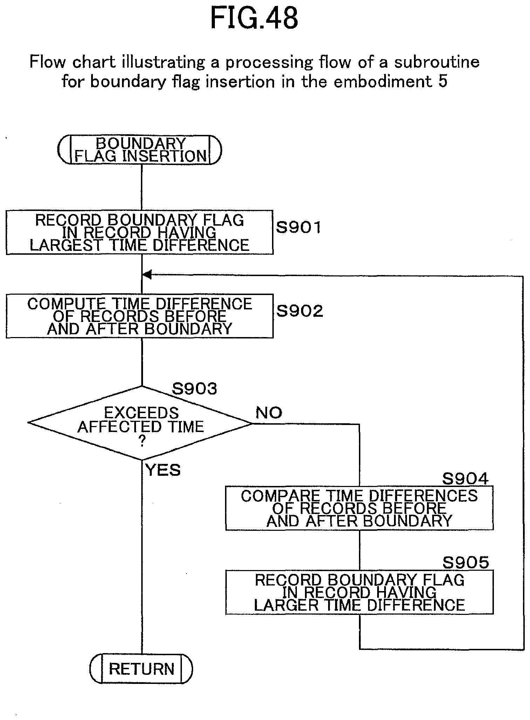

FIG. 48 is a flow chart illustrating a processing flow of a subroutine for boundary flag insertion in the embodiment 5. The subroutine for boundary flag insertion is a subroutine that inserts a flag indicating the boundary event into the boundary field of the group DB. The processing flow of the subroutine for group division in this embodiment will be described, by referring to FIG. 48.

The server CPU 22 extracts the record having the largest value recorded in the time difference field, from among the records that are the processing target, and records "boundary", which indicates a boundary event, in the flag field of this record having the largest value recorded in the time difference field (step S901). The server CPU 22 computes a time difference between the events before and after the boundary event (step S902). More particularly, the server CPU 22 computes the difference between the values recorded in the time fields of the records before and after the boundary event.

The server CPU 22 judges whether the computed time difference exceeds the affected time (step S903). In a case in which it is judged that the computed time difference is the affected time or less (NO in step S903), the server CPU 22 compares the time difference fields of the records before and after the boundary event (step S904). The server CPU 22 records "boundary", which indicates a boundary event, in the flag field of the record judged as having the larger value recorded in the time difference field (step S905). The server CPU 22 returns the process to step S902.

In a case in which it is judged that the computed time difference exceeds the affected time (YES in step S903), the server CPU 22 ends the process.

According to this embodiment, it is possible to provide the information processing system 10 having the short computation time, because it is possible to avoid processes having a large load, such as rearranging the DB.

### [Embodiment 6]

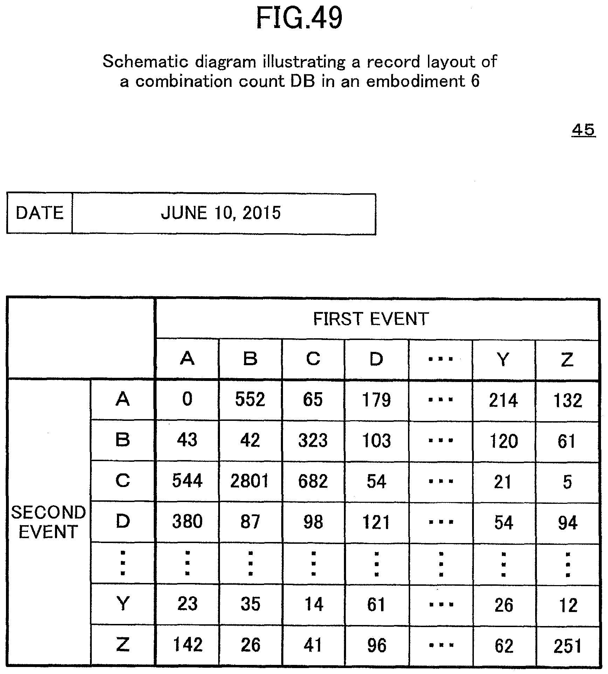

This embodiment relates to a configuration that creates a combination count DB 43 for 1 day for each day. FIG. 49 is a schematic diagram illustrating a record layout of the combination count DB 43 in the embodiment 6. A description of the parts in common with the embodiment 1 will be omitted.

The combination count DB 43 in this embodiment is a DB that records a count of the second event generated within a predetermined affected time, after the first event, for 1 day for each day. The combination count DB 43 includes a date field, a first event field, and a second event field. The date field is recorded with a related date. The combination count DB 43 in this embodiment is a DB that is added with the date field to the combination count DB 43 described in conjunction with FIG. 5. A separation of the dates is not limited to a time 0:00, and it is possible to appropriately select a time when a generation frequency of the events is small.

In this embodiment, the date field is similarly added to the generation number DB 44 described in conjunction with FIG. 7 and the combination time DB. Illustration of the generation number DB 44 and the combination time DB used in this embodiment will be omitted.

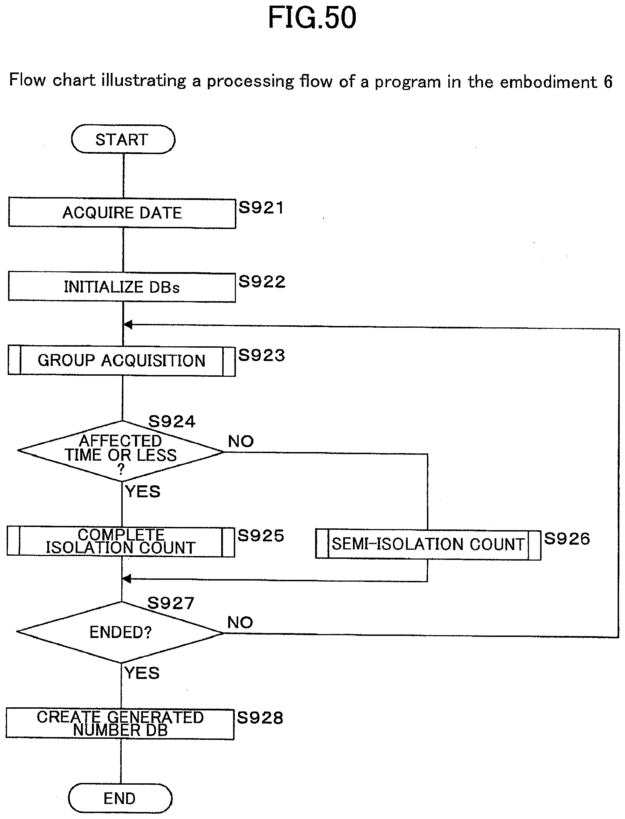

FIG. 50 is a flow chart illustrating a processing flow of a program in the embodiment 6. The program illustrated in FIG. 50 is a program that acquires the record from the time-series DB 42, and computes a count of a combination of 2 events generated within the affected time, for 1 day for each day. The processing flow of the program in this embodiment will be described, by referring to FIG. 50.

The server CPU 22 acquires a date on which the data is to be processed (step S921). In a case in which the process of the program in this embodiment is to be performed every day, it is desirable to set the date to a previous date. The server CPU 22 initializes the combination count DB 43 and the combination time DB (step S922). In other words, the server CPU 22 sets the values of each of the fields of the combination count DB 43 corresponding to the date acquired in step S921 to an initial value, which is 0. Further, the server CPU 22 deletes all of the records in the combination time DB corresponding to the date acquired in step S921.

The server CPU 22 boots a subroutine for group acquisition (step S923). The subroutine for group acquisition is a subroutine for acquiring the group of events each separated by the affected time or longer from 1 preceding event and from 1 subsequent event, from the data within the time-series DB 42. The subroutine for group acquisition uses the subroutine described in conjunction with FIG. 15, for example.

The server CPU 22 judges whether a time between the head event and the last event of the acquired group is the affected time or less (step S924). In a case in which it is judged that the time is the affected time or less (YES in step S924), the server CPU 22 boots a subroutine for complete isolation count (step S925). The subroutine for complete isolation count is a subroutine that records the combination of events within the complete isolation set in the combination count DB 43. The subroutine for complete isolation count uses the subroutine described in conjunction with FIG. 16, for example.

In a case in which the time is longer than the affected time NO in step S924), the server CPU 22 boots a subroutine for semi-isolation count (step S926). The subroutine for semi-isolation count is a subroutine that records the combination of events within the semi-isolation set in the combination count DB 43. The subroutine for semi-isolation count uses the subroutine described in conjunction with FIG. 17, for example.

After step S925 or step S926, the server CPU 22 judges whether analysis of the time-series DB 42 amounting to 1 day ended (step S927). The server CPU 22 acquires the end position of the analysis in step S502, as described above. In a case in which it is judged that the analysis has not ended (NO in step S927), the server CPU 22 returns the process to step S923. In a case in which it is judged that the analysis ended (YES in step S927), the server CPU 22 creates the generation number DB 44 by counting each of the events on the date acquired in step S921 (step S928). The server CPU 22 saves the combination count DB 43, the combination time DB, and the generation number DB 44 that are created by the processes described above, in the auxiliary storage device 24. The server CPU 22 thereafter ends the process.

According to this embodiment, the combination count DB 43, the combination time DB, and the generation number DB 44 for 1 day is created for each day and saved in the auxiliary storage device 24. Accordingly, the user who analyzes the data can easily analyze the data, by acquiring the data for an analyzing target date and read the acquired data into a general-purpose spreadsheet software or the like.

### [Embodiment 7]

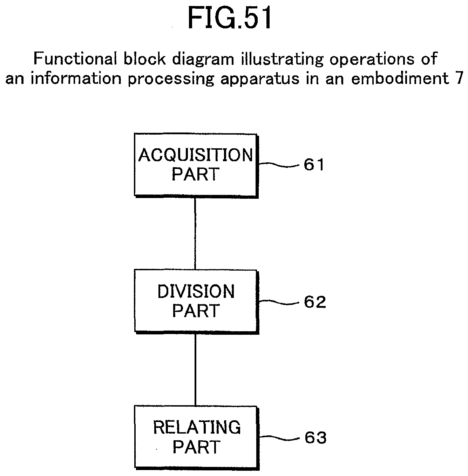

FIG. 51 is a functional block diagram illustrating an operation of the information processing apparatus in the embodiment 7. The information processing apparatus operates as follows based on the control performed by the CPU 12. An acquisition part 61 acquires time-series data including information related to a plurality of types of events and times related to the events, from the time-series DB 42. A division part 62 divides the time-series data into a plurality of groups having time intervals longer than the affected time, on the time base. A relating part 63 relates the combination of events included in the group, and the generation number, in a case in which a duration from the start time to the end time of the group is a related time or less.

### [Embodiment 8]

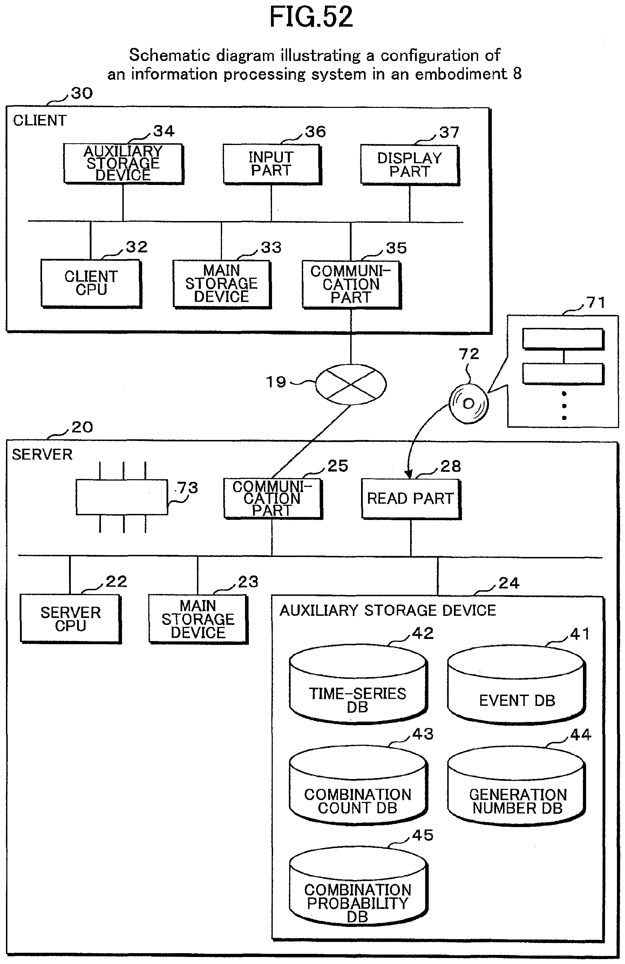

The embodiment 8 relates to a configuration that realizes the information processing system 10, by operating a combination of a general-purpose computer and a program 71. FIG. 52 is a schematic diagram illustrating configurations of the server 20 and the client 30 forming the information processing system 10 in the embodiment 8. The configuration of this embodiment will be described, by referring to FIG. 52. A description of the parts in common with the embodiment 1 will be omitted.

The server 20 includes the server CPU 22, the main storage device 23, the auxiliary storage device 24, the communication part 25, a read part 28, and a bus. The read part 28 is an optical disk reader, for example.

The program 71 is recorded in a portable recording medium 72. The server CPU 22 reads the program 71 via the read part 28, and saves the program 71 in the auxiliary storage device 24. In addition, the server CPU 22 may read the program 71 stored in a semiconductor memory 73, such as a flash memory or the like, implemented within an input device 10. Further, the CPU 12 may download the program 71 from another server computer that is not illustrated and is connected via the communication part 25 and the network 19, and save the downloaded program 71 in the auxiliary storage device 24.

A program 38 is installed as a control program of the server 20, and is loaded into the main storage device 23 and executed. Accordingly, the information processing system functions as the information processing system 10 described above.

Technical features (constituent elements) described in each of the embodiments may be mutually combined, and the combination may form new technical features.

The disclosed embodiments are merely examples in all respects, and are to be construed as being without limitations. The scope of the present invention is indicated by the claims and not in the meanings described above, and various changes, substitutions, and alterations could be made without departing from the spirit and scope of the invention.

(Appended Claim 1) A program which causes a computer to execute a process comprising:
acquiring time-series data including information related to types of events and times when the events are generated; and
dividing the time-series data into groups at positions where an interval between a time when one event is generated to a time when an event next to the one event is generated, is longer than a predetermined time by which the events are regarded as having causality therebetween.

(Appended Claim 2) The program according to Appended Claim 1, which causes the computer to execute the process comprising:
relating combinations of events included in a group and a generation number of the combinations, for each of the groups.

(Appended Claim 3) The program according to Appended Claim 1 or Appended Claim 2, which causes the computer to execute the process comprising:
dividing a second group on a time base from the group, in a case in which a duration from a start time to an end time of the group is longer than the predetermined time, wherein the second group includes events from a head event of the group up to an event before a boundary event for which a duration from a time when the head event is generated to a time when an event before a boundary event within the group is generated is shorter than the predetermined time, and an interval from the time when the event before the boundary event is generated to a time when an event next to the boundary event is generated is longer than the predetermined time;
relating combinations of the events included in the second group and a generation number of said combinations; and
relating combinations of events generated at times in a range of the predetermined time from the time when the boundary event is generated, and the boundary event, and a generation number of said combinations.

(Appended Claim 4) The program according to Appended Claim 3, wherein the boundary event is determined based on time intervals between the boundary event and adjacent events on the time base.

(Appended Claim 5) The program according to Appended Claim 3 or Appended Claim 4, wherein the boundary events are an event determined based on a time interval between adjacent events on the time base, and an event consecutive to said event on the time base.

(Appended Claim 6) The program according to any one of Appended Claim 3 to Appended Claim 5, wherein the boundary events include an event having a longest time interval from an adjacent event on the time base within the group.

(Appended Claim 7) The program according to Appended Claim 1, which causes the computer to execute the process comprising:
dividing, in a case in which a duration from a start time to an end time of the group is longer than the predetermined time, the group into a plurality of second groups having intervals longer than the predetermined time with events that are adjacent via a predetermined number of boundary events on a time base;
repeating, in a case in which a duration from a start time to an end time of the second group is longer than the predetermined time, subdividing the second group into a plurality of second groups having the intervals longer than the predetermined time with events that are adjacent via the predetermined number of boundary events on the time base by increasing the predetermined number;
relating combinations of the events included in the second group in which the duration from the start time to the end time is shorter than the predetermined time, and a generation number of said combinations; and
relating combinations of events generated at times in a range of the predetermined time from the time when the boundary event is generated, and the boundary event, and a generation number of said combinations.

(Appended Claim 8) The program according to Appended Claim 7, wherein the predetermined number has an initial value that is a number of minimum time intervals exceeding the predetermined time by successively adding time intervals between each event within the group and an adjacent event on the time base, starting from a longest time interval.

(Appended Claim 9) The program according to Appended Claim 1, which causes the computer to execute the process comprising:
dividing, in a case in which a duration from a start time to an end time of the group is longer than the predetermined time, the group into a second group on a time base, by a boundary event having a largest interval with an event that is adjacent to the boundary event on a time base within the group;
repeating, in a case in which a duration from a start time to an end time of the second group is longer than the predetermined time, adding the event that is adjacent to the boundary event on the time base to boundary events;
relating combinations of the events included in the second group in which the duration from the start time to the end time is shorter than the predetermined time, and a generation number of said combinations; and
relating combinations of events generated at times in a range of the predetermined time from the time when the boundary event is generated, and the boundary event, and a generation number of said combinations.

(Appended Claim 10) program according to Appended Claim 1, which causes the computer to execute the process comprising:
dividing, in a case in which a duration from a start time to an end time of the group is longer than the predetermined time, the group into a second group on a time base, by a boundary event having a largest interval with an event that is adjacent to the boundary event on a time base within the group;
repeating, in a case in which a duration from a start time to an end time of the second group is longer than the predetermined time, subdividing the second group into a plurality of second groups on the time base, by a boundary event having a largest interval with an event that is adjacent to the boundary event on the time base within the second group;
relating combinations of the events included in the second group in which the duration from the start time to the end time is shorter than the predetermined time, and a generation number of said combinations; and
relating combinations of events generated at times in a range of the predetermined time from the time when the boundary event is generated, and the boundary event, and a generation number of said combinations.

(Appended Claim 11) The program according to any one of Appended Claim 1 to Appended Claim 10, which causes the computer to execute the process comprising:

(Appended Claim 12) The program according to any one of Appended Claim 2 to Appended Claim 11, wherein the combinations are combinations of one event and another event generated after the one event.

(Appended Claim 13) The program according to any one of Appended Claim 2 to Appended Claim 12, which causes the computer to execute the process comprising:
computing a sum of the generation number for each of the combinations.

(Appended Claim 14) The program according to Appended Claim 13, which causes the computer to execute the process comprising:
computing a number of the events included in the time-series data for each of the types; and
computing a ratio of a number of events of one type to the sum corresponding to said event and another type of event generated after said event.

(Appended Claim 15) The program according to any one of Appended Claim 1 to Appended Claim 14, wherein the time is a time when generation of the event is detected.

(Appended Claim 16) The program according to any one of Appended Claim 1 to Appended Claim 14, wherein the time is a time when generation of the event is recorded.

(Appended Claim 17) An information processing method which causes a computer to execute a process comprising:
acquiring time-series data including information related to types of events and times when the events are generated; and
dividing the time-series data into groups at positions where an interval between a time when one event is generated to a time when an event next to the one event is generated, is longer than a predetermined time by which the events are regarded as having causality therebetween.

(Appended Claim 18) An information processing processing apparatus comprising:
an acquisition part configured to acquire time-series data including information related to types of events and times when the events are generated; and
a division part configured to divide the time-series data into groups at positions where an interval between a time when one event is generated to a time when an event next to the one event is generated, is longer than a predetermined time by which the events are regarded as having causality therebetween.

### DESCRIPTION OF THE REFERENCE NUMERALS

- 10: Information Processing System
- 12: Sensor Control Device
- 13: Door Sensor
- 14: Camera
- 15: Bed Sensor
- 16: Biometric Sensor
- 17: Portable Information Device
- 19: Network
- 20: Server
- 22: Server CPU
- 23: Main Storage Device
- 24: Auxiliary Storage Device
- 25: Communication Part
- 28: Read Part
- 30: Client
- 32: Client CPU
- 36: Input Part
- 37: Display Part
- 41: Event DB
- 42: Time-Series DB
- 43: Combination Count DB
- 44: Generation Number DB
- 45: Combination Probability DB
- 51: List Column
- 52: Analysis Period
- 53: ID Column
- 55: Data Column
- 61: Acquisition Part
- 62: Division Part
- 63: Count Part
- 71: Program
- 72: Portable Recording Medium
- 73: Semiconductor Memory

## Claims

1. A program which causes a computer to execute a process comprising:
acquiring time-series data including information related to types of events and times when the events are generated; and
dividing the time-series data into groups at positions where an interval between a time when one event is generated to a time when an event next to the one event is generated, is longer than a predetermined time by which the events are regarded as having causality therebetween.

2. The program according to claim 1, which causes the computer to execute the process comprising:
relating combinations of events included in a group and a generation number of the combinations, for each of the groups.

3. The program according to claim 1 or claim 2, which causes the computer to execute the process comprising:
dividing a second group on a time base from the group, in a case in which a duration from a start time to an end time of the group is longer than the predetermined time, wherein the second group includes events from a head event of the group up to an event before a boundary event for which a duration from a time when the head event is generated to a time when an event before a boundary event within the group is generated is shorter than the predetermined time, and an interval from the time when the event before the boundary event is generated to a time when an event next to the boundary event is generated is longer than the predetermined time;
relating combinations of the events included in the second group and a generation number of said combinations; and
relating combinations of events generated at times in a range of the predetermined time from the time when the boundary event is generated, and the boundary event, and a generation number of said combinations.

4. The program according to claim 1 or claim 2, which causes the computer to execute the process comprising:
dividing, in a case in which a duration from a start time to an end time of the group is longer than the predetermined time, the group into a plurality of second groups having intervals longer than the predetermined time with events that are adjacent via a predetermined number of boundary events on a time base;
repeating, in a case in which a duration from a start time to an end time of the second group is longer than the predetermined time, subdividing the second group into a plurality of second groups having the intervals longer than the predetermined time with events that are adjacent via the predetermined number of boundary events on the time base by increasing the predetermined number;
relating combinations of the events included in the second group in which the duration from the start time to the end time is shorter than the predetermined time, and a generation number of said combinations; and
relating combinations of events generated at times in a range of the predetermined time from the time when the boundary event is generated, and the boundary event, and a generation number of said combinations.

5. The program according to claim 4, wherein the predetermined number has an initial value that is a number of minimum time intervals exceeding the predetermined time by successively adding time intervals between each event within the group and an adjacent event on the time base, starting from a longest time interval.

6. The program according to any one of claim 2 to claim 5, which causes the computer to execute the process comprising:
computing a sum of the generation number for each of the combinations.

7. The program according to claim 6, which causes the computer to execute the process comprising:
computing a number of the events included in the time-series data for each of the types; and
computing a ratio of a number of events of one type to the sum corresponding to said event and another type of event generated after said event.

8. An information processing method which causes a computer to execute a process comprising:
acquiring time-series data including information related to types of events and times when the events are generated; and
dividing the time-series data into groups at positions where an interval between a time when one event is generated to a time when an event next to the one event is generated, is longer than a predetermined time by which the events are regarded as having causality therebetween.

9. An information processing processing apparatus comprising:
an acquisition part configured to acquire time-series data including information related to types of events and times when the events are generated; and
a division part configured to divide the time-series data into groups at positions where an interval between a time when one event is generated to a time when an event next to the one event is generated, is longer than a predetermined time by which the events are regarded as having causality therebetween.
